(19) Europäisches Patentamt / European Patent Office / Office européen des brevets

(11) **EP 2 630 911 A1**

(12) **EUROPEAN PATENT APPLICATION**

(43) Date of publication:
**28.08.2013 Bulletin 2013/35**

(51) Int Cl.:
*A61B 5/022* (2006.01)　　*A61B 5/00* (2006.01)

(21) Application number: **13156582.2**

(22) Date of filing: **25.02.2013**

| | |
|---|---|
| (84) Designated Contracting States:<br>**AL AT BE BG CH CY CZ DE DK EE ES FI FR GB GR HR HU IE IS IT LI LT LU LV MC MK MT NL NO PL PT RO RS SE SI SK SM TR**<br>Designated Extension States:<br>**BA ME**<br><br>(30) Priority: **23.02.2012 JP 2012037479**<br>　　　　　　**14.11.2012 JP 2012250562** | (71) Applicant: **Tanita Corporation Tokyo 174 (JP)**<br><br>(72) Inventor: **Hotta, Junji Itabashi-ku, Tokyo 174-8630 (JP)**<br><br>(74) Representative: **Grünecker, Kinkeldey, Stockmair & Schwanhäusser Leopoldstrasse 4 80802 München (DE)** |

(54) **Blood pressure measurement apparatus and blood pressure measurement method**

(57)　A blood pressure measurement apparatus includes a blood pressure measurement unit, a bio-information acquisition unit, a depth calculation unit, a storage unit and a decision unit. The bio-information acquisition unit continuously acquires bio-information of a subject. The depth calculation unit repeatedly calculates depth indices each indicating a sleep stage of the subject on the basis of the acquired bio-information. The storage unit stores the calculated depth indices. The decision unit decides whether the sleep stage of the subject in sleep satisfies a starting condition, on the basis of a first depth index stored in the storage unit and a second depth index calculated by the depth calculation unit. The blood pressure measurement unit measures blood pressure data of the subject when the sleep stage is decided to satisfy the starting condition.

FIG.2

EP 2 630 911 A1

## Description

This application is based on Japanese patent application No.2012-037479 and Japanese patent application No.2012-250562, the contents of which are incorporated hereinto by reference.

BACKGROUND

TECHNICAL FIELD

The present invention relates to a blood pressure measurement apparatus that measures blood pressure during sleep, and to a method of measuring blood pressure.

RELATED ART

From the viewpoint of healthcare management, it is important to accurately recognize one's blood pressure. The blood pressure readily fluctuates owing to just a gentle physical movement after waking up or some mental factors, and hence it is desirable to measure the blood pressure while the subject is asleep in order to know the true level of the subject's blood pressure. The blood pressure during a deep sleep is called basal blood pressure, which is useful for properly performing the healthcare management. The sleep condition of a human can be broadly classified into REM sleep and non-REM sleep, and the non-REM sleep is further classified into 2 to 4 stages depending on the depth of sleep. It is known that, in general, human beings alternately repeat the REM sleep and the non-REM sleep at an ultradian rhythm in periods of 60 minutes to 90 minutes. Accordingly, some techniques have been developed to measure blood pressure in association with the depth of sleep, by attaching an apparatus having various sensors to the subject when the subject goes to bed.

For example, JP-A-2007-229238 discloses a blood pressure measurement apparatus configured to determine a level of the depth of sleep at a certain point during the sleep, on the basis of a ratio of a pulse wave period at that point to a pulse wave period immediately after the subject falls asleep, and to measure the subject's blood pressure when the level of the depth satisfies a predetermined condition. Preferable conditions for measuring the blood pressure are, according to this document, when the depth of sleep reaches a fourth level, which is the deepest of the non-REM sleep, and also when the level of the depth suddenly turns shallower.

In addition, JP-A-2006-212218 discloses a healthcare management apparatus configured to measure a pulse wave propagation time of the subject in sleep, corresponding to a time for a pulse wave to reach a peripheral vein from the heart, by a combination of an electrocardiography and pulse wave measurement, to thereby estimate the blood pressure on the basis of the reciprocal of the pulse wave propagation time. The apparatus calculates an index of a low frequency region and an index of a high frequency region through analysis of the waveform of blood pressure levels that fluctuate with time, and compares the magnitude between each of the indices and a threshold to thereby determine the depth of sleep.

[Patent Document 1] JP-A-2007-229238
[Patent Document 2] JP-A-2006-212218

SUMMARY

PROBLEM TO BE SOLVED BY THE INVENTION

However, the pulse wave period during sleep largely varies depending on factors such as the age and physical constitution of the subject, which makes it difficult for the blood pressure measurement apparatus according to JP-A-2007-229238 to accurately determine the depth of sleep. For example, from a senior aged subject, or from a subject whose sleep is naturally shallow, a visible difference may not be observed between the pulse wave period immediately after falling asleep and the pulse wave period during a non-REM sleep. In such a case, the depth of sleep that satisfies the predetermined measurement condition may fail to be detected during the entire sleeping process of the subject. Thus, the measurement accuracy largely varies among individual subjects.

In the healthcare management apparatus JP-A-2006-212218, some attempts for reducing individual differences in calculation accuracy of the blood pressure are made, such as measuring in advance a coefficient for multiplying the reciprocal of the pulse wave propagation time to calculate the blood pressure and the threshold for determining the depth of sleep, with respect to each subject for calibration. However, since the healthcare management apparatus according to JP-A-2006-212218 is designed to estimate the blood pressure from the pulse wave propagation time, the calculation accuracy of the blood pressure is not sufficiently high. In addition, since the apparatus according to JP-A-2006-212218 is designed to analyze the subject's sleep condition through analysis of the waveform of the blood pressure that fluctuates with time, the pulse wave propagation time and the blood pressure have to be continuously measured during the entire sleeping process of the subject, in order to estimate the blood pressure during a non-REM sleep. Accordingly, the apparatus according to JP-A-2006-212218 imposes a trouble on the subject thus to disturb sound sleep, and is hence unable to measure stabilized blood pressure of the subject in sleep. Thus, the apparatuses according to JP-A-2007-229238 and JP-A-2006-212218 are not suitable for accurately measuring the blood pressure while the subject is asleep.

The present invention has been accomplished in view of the foregoing problem, and provides an apparatus and a method that allow truly stabilized blood pressure of a subject to be accurately measured irrespective of the age and sleep characteristics of the subject.

SOLUTION TO PROBLEM

In one embodiment, there is provided a blood pressure measurement apparatus including a blood pressure measurement unit that measures blood pressure of a subject; a bio-information acquisition unit that continuously acquires bio-information of the subject; a depth calculation unit that repeatedly calculates a depth index indicating a sleep stage of the subject on the basis of the acquired bio-information; a storage unit in which the calculated depth index is stored; and a decision unit that decides whether the sleep stage of the subject in sleep satisfies a predetermined condition, on the basis of a first depth index stored in the storage unit and a second depth index calculated by the depth calculation unit, and the blood pressure measurement unit measures the blood pressure when the decision unit decides that the sleep stage satisfies the condition.

In another embodiment, there is provided a method of measuring blood pressure, including preliminarily acquiring first bio-information of a subject in sleep thereby calculating a first depth index indicating a sleep stage of the subject; storing the calculated first depth index; acquiring second bio-information of the subject in sleep thereby calculating a second index indicating the sleep stage, the second bio-information being different from the first bio-information; deciding whether the sleep stage satisfies a predetermined condition on the basis of the stored first depth index and the calculated second depth index; and repeating the acquiring, the calculating and the deciding, and measuring blood pressure of the subject upon deciding that the sleep stage satisfies the condition.

In the foregoing arrangement, the depth index indicating the sleep stage of the subject is stored in advance, and the time point for measuring the blood pressure is determined on the basis of the stored depth index and the depth index of the subject in sleep. Therefore, the measurement result of the blood pressure is prevented from fluctuating among the individual subjects. The foregoing arrangement also eliminates the need to continuously measure the blood pressure during the entire sleep process of the subject, thereby saving the subject from disturbance to sleep.

In still another embodiment, there is provided a blood pressure measurement apparatus including a blood pressure measurement unit that measures blood pressure of a subject; a bio-information acquisition unit that continuously acquires bio-information of the subject; a depth calculation unit that calculates a sleep stage of the subject on the basis of the acquired bio-information; a storage unit in which a reference sleep stage indicating a predetermined sleep stage is stored; and a decision unit that decides whether the sleep stage of the subject in sleep has remained in the reference sleep stage for a predetermined time, on the basis of the reference sleep stage stored in the storage unit and the sleep stage calculated by the depth calculation unit. The blood pressure measurement unit measures the blood pressure when the decision unit decides that the sleep stage of the subject in sleep has remained in the reference sleep stage for the predetermined time.

In still another embodiment, there is provided a method of measuring blood pressure including measuring blood pressure of a subject; continuously acquiring bio-information of the subject; calculating a sleep stage of the subject on the basis of the acquired bio-information; and deciding whether the sleep stage of the subject in sleep has remained for a predetermined time in a reference sleep stage indicating a predetermined sleep stage, on the basis of the reference sleep stage and the sleep stage calculated in the step of calculating. The measuring of blood pressure includes measuring the blood pressure upon deciding that the sleep stage of the subject in sleep has remained in the reference sleep stage for the predetermined time.

By the foregoing arrangement, the measuring operation of the blood pressure is activated in the case where the sleep stage has remained in a predetermined state (reference sleep stage) for a predetermined time. In other words, in the case where the sleep stage has remained in the predetermined state for a period shorter than the predetermined time, for example only momentaneously, the measurement of the blood pressure is not thereby activated. Such an arrangement allows the sleep stage of the subject to be accurately decided, thereby enabling truly stabilized blood pressure of the subject to be accurately measured.

Thus, the apparatus and the method arranged as above allow truly stabilized blood pressure of a subject to be accurately measured irrespective of the age and sleep characteristics of the subject.

BRIEF DESCRIPTION OF THE DRAWINGS

The above and other objects, advantages and features of the present invention will be more apparent from the following description taken in conjunction with the accompanying drawings, in which:

Fig. 1 is a perspective view showing a use of a blood pressure measurement apparatus according to a first embodiment of the present invention;

Fig. 2 is a block diagram showing a configuration of the blood pressure measurement apparatus according to the first embodiment;

Figs. 3A and 3B are schematic cross-sectional views of a blood pressure measurement unit attached to the arm portion of a subject, for example the wrist, Fig. 3B showing a state where the blood pressure measurement unit is oppressing the arm portion;

Fig. 4 is a flowchart showing a general process of a blood pressure measurement method;

Fig. 5 is a flowchart showing details of a preliminary process;

Fig. 6 is a flowchart showing details of a main measurement process;

Fig. 7 is a graph showing sleep characteristics of a subject obtained through a sampling measurement;

Fig. 8 is a graph showing sleep characteristics of the subject obtained through a main measurement process;

Fig. 9 is a block diagram showing a configuration of a blood pressure measurement apparatus according to a second embodiment; and

Fig. 10 is a flowchart showing a blood pressure measurement process according to a variation of the embodiments.

DETAILED DESCRIPTION

The invention will be now described herein with reference to illustrative embodiments. Those skilled in the art will recognize that many alternative embodiments can be accomplished using the teachings of the present invention and that the invention is not limited to the embodiments illustrated for explanatory purposes.

Hereafter, some embodiments of the present invention will be described referring to the drawings. In all the drawings, the same constituents will be given the same numeral, and the description thereof will not be repeated.

FIRST EMBODIMENT

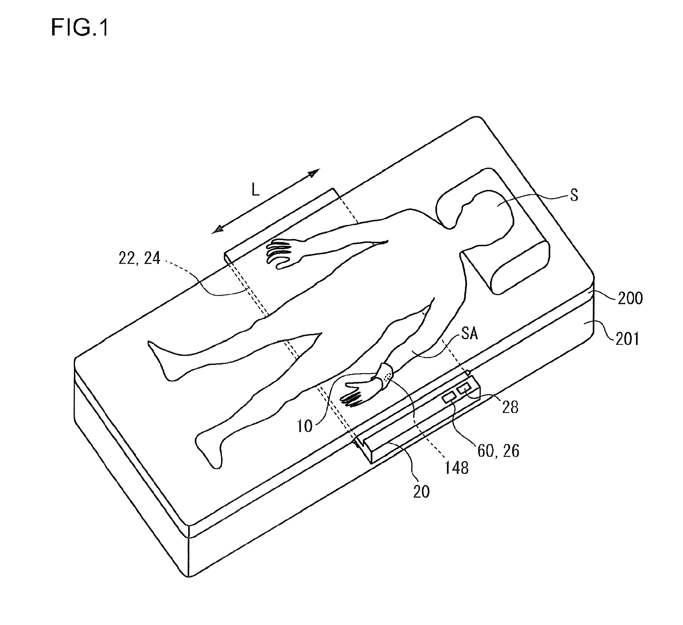

Fig. 1 is a perspective view showing a use of a blood pressure measurement apparatus 100 according to a first embodiment of the present invention.

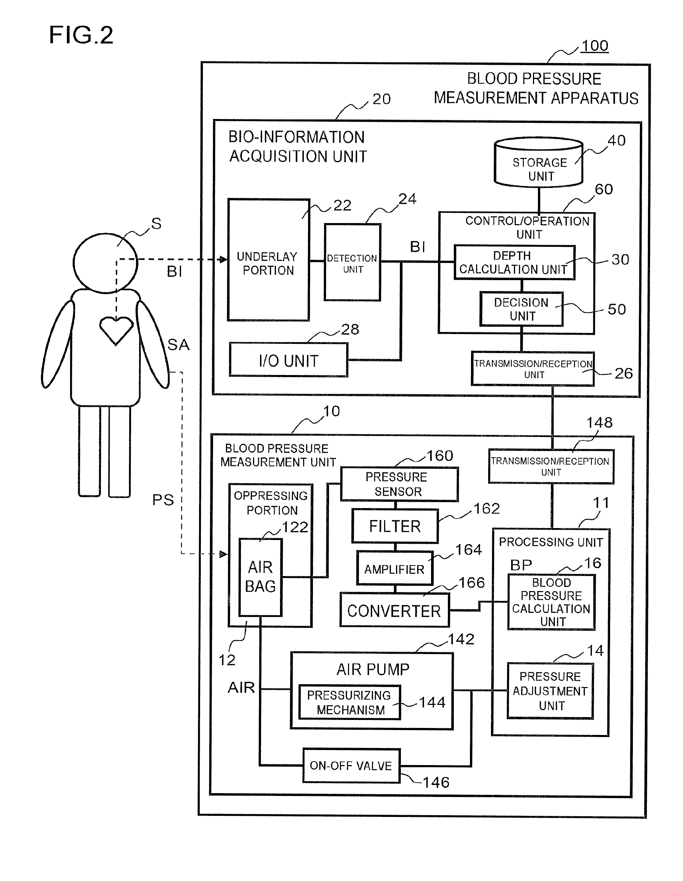

Fig. 2 is a block diagram showing a configuration of the blood pressure measurement apparatus 100 according to the first embodiment.

First, the outline of this embodiment will be described.

The blood pressure measurement apparatus 100 according to this embodiment includes a blood pressure measurement unit 10, a bio-information acquisition unit 20, a depth calculation unit 30, a storage unit 40, and a decision unit 50.

The blood pressure measurement unit 10 is configured to measure blood pressure of a subject S. The bio-information acquisition unit 20 is configured to continuously acquire bio-information BI of the subject S. The depth calculation unit 30 is configured to repeatedly calculate a depth index $\alpha$ indicating a sleep stage of the subject S, on the basis of the acquired bio-information BI. The storage unit 40 serves to store therein the calculated depth index $\alpha$. The decision unit 50 is configured to decide whether the sleep stage of the subject S satisfies a predetermined condition (starting condition), on the basis of a first depth index $\alpha2$ stored in the storage unit 40 and a second depth index $\alpha$ calculated by the depth calculation unit 30.

The depth calculation unit 30 and the decision unit 50 constitute a control/operation unit 60 composed of a central processing unit (CPU).

The blood pressure measurement unit 10 according to this embodiment measures the blood pressure when the decision unit 50 decides that the sleep stage satisfies the starting condition.

Hereunder, this embodiment will be described in further details.

The blood pressure measurement apparatus 100 according to this embodiment is intended for use by an individual user to measure blood pressure at home. The blood pressure measurement apparatus 100 continuously or intermittently acquires the bio-information BI from the subject S in sleep with the bio-information acquisition unit 20 to thereby calculate the sleep stage, and activates the blood pressure measurement unit 10 to measure the blood pressure when the sleep stage satisfies the predetermined starting condition, which will be subsequently described. Examples of the bio-information BI that may be acquired by the bio-information acquisition unit 20 include body motion, pulse wave, brain wave, body temperature, number of breaths (respiratory rate), number of pulses (pulse rate), and number of heartbeats (heart rate). Two or more of those may be measured for the calculation of the sleep stage. In this embodiment, the body motion is adopted as the bio-information BI to be measured, and the pulse wave is adopted in the second embodiment to be subsequently described. The measurement methods of other types of bio-information BI are known and hence detailed description will not be given.

The bio-information acquisition unit 20 according to this embodiment shown in Fig. 1 includes an underlay portion 22 spread under the lying subject S, and a detection unit 24 that acquires body motion information MV as the bio-information BI, the body motion information MV indicating the body motion of the subject S on the basis of the self weight of the subject S imposed on the underlay portion 22. The body motion information MV is composed of waveform data representing a profile of periodic body motions of the subject S, such as the breaths and heart beats.

The underlay portion 22 is a flat bag in which a non-compressible fluid such as water or air is enclosed. The subject S may lie directly on the underlay portion 22. Alternatively, the underlay portion 22 may be interleaved, as shown in Fig. 1, between a thin bed pad 200 and a thick mattress 201 and the subject S may lie on the bed pad 200. Typically, the subject S lies in a supine position.

The longitudinal size L of the underlay portion 22 along the body height direction of the subject S is shorter than the height of the subject S. The widthwise size of the underlay portion 22 (orthogonal to the longitudinal size L) is wider than the width of the body trunk of the subject S, and generally the same as, for example, a width of a single bed. With such a configuration, when the subject S lies on the bed with the head and distal portions of the legs placed on the bed pad 200, a part of the self weight of the subject S, more specifically the self weight of the chest is imposed on the underlay portion 22. Loading on the underlay portion 22 the weight of a part of the body, instead of the entire body, of the subject S including the chest which periodically deforms with the heartbeats and breaths allows the load imposed on the underlay portion 22 to vary with the body motion such as the heart beats and breaths. Therefore, the body motion of the subject S can be detected by measuring pressure fluctuation of the fluid enclosed in the underlay portion 22 with the detection unit 24.

The detection unit 24 includes a piezoelectric conversion element, such as a condenser microphone, that converts pressure (acoustic pressure) into an electrical signal, and is disposed in contact with the bag body of the underlay portion 22 and electrically connected to the underlay portion 22.

The bio-information acquisition unit 20 includes an input/output unit (hereinafter, I/O unit) 28. The I/O unit 28 includes an operating button by which an instruction to start or stop the detection of the body motion information MV is received from the subject S. The I/O unit 28 also includes a display unit or a data recording unit. The blood pressure (systolic blood pressure and diastolic blood pressure) measured by the blood pressure measurement unit 10 and the number of heartbeats (number of pulses) may be displayed on the display unit, or may be recorded in a portable storage medium through the data recording unit.

The bio-information acquisition unit 20 according to this embodiment is configured to measure variation with time of the self weight of the lying subject S on the basis of the pressure of the fluid enclosed in the underlay portion 22, without oppressing the subject S. Accordingly, the subject S is not subjected to a load when the detection unit 24 acquires the bio-information BI. Therefore the subject S can continuously sleep undisturbed, despite the detection unit 24 acquiring the bio-information BI from the subject S continuously or a multitude of times. In this embodiment, it is preferable to set the bio-information acquisition unit 20 to acquire the bio-information BI (body motion information MV) at a sampling frequency of 1 Hz or higher.

As shown in Fig. 2, the bio-information BI (body motion information MV) acquired by the bio-information acquisition unit 20 from the subject S is transmitted to the depth calculation unit 30 in the control/operation unit 60.

The depth calculation unit 30 calculates the depth index $\alpha$ indicating the sleep stage of the subject S, on the basis of the bio-information BI (body motion information MV). The sleep stage is a quantified value of the depth of sleep of the subject S. Normally a larger value is used to express a deeper sleep, however a shallower sleep may be expressed by a larger value. The number of sleep stages is not specifically limited, but may be 3 to 10 stages, for example. More specifically, the depth of sleep may be set in 6 stages, which are awake (resting), REM sleep, and levels 1 to 4 of non-REM sleep. Setting a multiple of sleep stages enables the sleep stages to substantially represent continuous values.

The depth index $\alpha$ is data having a positive or negative correlation with the sleep stage. The depth index $\alpha$ and the sleep stage may be mutually convertible, or unilaterally convertible from the depth index $\alpha$ to the sleep stage. The depth calculation unit 30 includes a filter that cuts a frequency component outside an upper and a lower limit of the frequency region of the body motion, an amplifier that amplifies the waveform of the body motion information MV detected by the detection unit 24, and a frequency analyzer that performs frequency analysis in a predetermined frequency region. The depth calculation unit 30 divides the waveform data of the body motion information MV into epochs of a predetermined length, and performs the frequency analysis of the body motion information MV with respect to each of the epochs. The length of the epoch is not specifically limited, but it is preferable that the epoch is sufficiently shorter (for example, 30 seconds) than the duration of the respective sleep conditions, such as the REM sleep and the levels 1 to 4 of the non-REM sleep.

The depth index $\alpha$ may represent, for example, a period of the principal component of a relatively low frequency region such as 0.25 to 1 Hz indicating the breath waveform (respiratory rate) of the subject S, or a period of the principal component of a relatively high frequency region such as 0.75 to 2 Hz indicating the pulse wave (pulse rate) of the subject S. More broadly, for example, the power level of the power spectrum of the low frequency region or the high frequency region, numerical data such as a ratio of the power level of the low frequency region or the high frequency region to the power level of the overall frequency region, or waveform data of the power spectrum may be employed as the depth index $\alpha$.

The depth calculation unit 30 repeatedly calculates the depth index $\alpha$ of the subject S in sleep. The decision unit 50 compares, on the basis of the repeatedly calculated depth indices $\alpha$, between the past depth index (first depth index $\alpha2$) obtained in advance from the subject S through the sampling measurement and a newly calculated second depth index $\alpha$ from a main measurement, to thereby decide the starting timing of the blood pressure measurement. The main measurement refers to acquiring the bio-information BI and calculating the depth index $\alpha$ at the time point of the sleep when the blood pressure is to be measured. To be more detailed, the decision unit 50 decides that the sleep stage of an epoch satisfies the predetermined starting condition, when the depth index $\alpha$ calculated by the depth calculation unit 30 reaches close to the first depth index $\alpha2$ stored in the storage unit 40. More specifically, the decision unit 50 according to this embodiment decides that the sleep condition of the subject S satisfies the starting condition when the divergence between the first depth index $\alpha2$ and the second depth index $\alpha$ remains below the threshold through a predetermined number of successive epochs. Such an arrangement prevents erroneous operation of the blood pressure measurement unit 10 originating from fluctuation of the body motion information MV.

The first depth index $\alpha2$ adopted in this embodiment is the depth index that indicates the deepest sleep stage of the subject S among the plurality of depth indices $\alpha$ each calculated on the basis of the bio-information BI (body motion information MV) acquired at different time points. Here, the expression "different time points" includes different time points during a single sleep session, as well as different time points in a plurality of sleep sessions of different days. The blood pressure measurement apparatus 100 according to this embodiment is configured to store the depth index (first depth index $\alpha2$) of the subject S corresponding to the depth level 4 of the non-REM sleep, in the storage unit 40. In this embodiment, a larger value of the sleep stage indicates a deeper sleep. The sleep stage may also be calculated during a resting state before the subject S falls asleep, in addition to during the sleep. To make the first depth index $\alpha2$ indicate the deepest sleep stage of the subject S, for example the depth index corresponding to a maximum value of the sleep stage during a single sleep session is set as the first depth index $\alpha2$, in the case where the larger value is used to indicate the deeper sleep. Hereafter, the deeper sleep will be indicated by the larger value of the sleep stage, unless otherwise noted. Alternatively, the first depth index $\alpha2$ may be the average of several largest values of the sleep stage in a sleep session, or the average of the respective largest values of a plurality of sleep sessions. To further improve the measurement accuracy, in the case where the largest value of the sleep stage is significantly different from the second largest value, the largest value may be construed as an error and the second largest value may be adopted as the first depth index $\alpha2$.

The storage unit 40 also contains a depth index calculated on the basis of the bio-information BI acquired in the resting state before the sleep (hereinafter, at-rest index $\alpha1$), and a threshold coefficient $\gamma$, in addition to the first depth index $\alpha2$ indicating the deepest sleep stage of the subject S. The sleep stage indicated by the at-rest index $\alpha1$ has a smaller value than the sleep stage indicated by the first depth index $\alpha2$. The decision unit 50 decides that the starting condition for the blood pressure measurement is satisfied in the case where the difference between the second depth index $\alpha$ calculated by the depth calculation unit 30 and the first depth index $\alpha2$ stored in the storage unit 40 has been repeatedly below the threshold for a predetermined length of time. The specific decision method of the starting condition will be subsequently described. The storage unit 40 according to this embodiment is a non-volatile memory. Accordingly, when the at-rest index $\alpha1$ and the first depth index $\alpha2$ obtained in the preliminary process are stored in the storage unit 40 and then the blood pressure measurement apparatus 100 is turned off, such recorded data is maintained. The at-rest index $\alpha1$ and the first depth index $\alpha2$ recorded earlier can be utilized when the blood pressure measurement apparatus 100 is again turned on at a later date, for deciding the starting condition for the blood pressure measurement.

The bio-information BI acquired in the preliminary measurement for calculating the at-rest index $\alpha1$ and the first depth index $\alpha2$ to be stored in the storage unit 40 may be of the same type or of a different type from the bio-information BI acquired in the main measurement. For example, the at-rest index $\alpha1$, the first depth index $\alpha2$, and the second depth index $\alpha$ may be calculated on the basis of the minimum respiratory rate in both of the preliminary measurement and the main measurement. Alternatively, the at-rest index $\alpha1$ and the first depth index $\alpha2$ may be calculated on the basis of the minimum pulse rate of the subject S in the preliminary measurement, and the second depth index $\alpha$ may be calculated on the basis of the minimum respiratory rate in the main measurement.

Upon deciding that the starting condition for the blood pressure measurement is satisfied, the decision unit 50 transmits a start signal to a pressure adjustment unit 14 (see Fig. 2) of the blood pressure measurement unit 10, so as to activate an air pump 142 and an on-off valve 146. With such an arrangement, the predetermined sleep stage of the subject S (in this embodiment, level 4 of the non-REM sleep) can be accurately recognized irrespective of the sleep characteristics of each individual subject S.

As shown in Fig. 1, the blood pressure measurement unit 10 includes a transmission/reception unit 148, and the bio-information acquisition unit 20 includes a transmission/reception unit 26. The transmission/reception units 26, 148 are wireless interfaces capable of performing bidirectional communication. In the bio-information acquisition unit 20, the control/operation unit 60 (depth calculation unit 30, decision unit 50) and the storage unit 40 are integrally incorporated.

The blood pressure measurement unit 10 actually measures the pressure in the blood vessel of the subject S. The measurement process of the blood pressure is broadly classified into invasive methods and non-invasive methods, the latter of which are preferable from the viewpoint of reducing the burden on the subject S. Well known non-invasive blood pressure measurement methods include auscultation by measuring a Korotkoff sound (Korotkoff method) and a pulse wave method in which the pulse wave formed by propagation of heartbeats through the arterial wall is measured. The Korotkoff sound and the pulse wave are measured by oppressing a portion of the upper limb of the subject S, such as upper arm, wrist, or fingertip. Examples of known pulse wave method include oscillometry, tonometry, and volume compensation, each of which adopts a different measurement method. Either the auscultation or the pulse wave method may be employed in the blood pressure measurement unit 10. Both methods may be employed in combination for the blood pressure measurement.

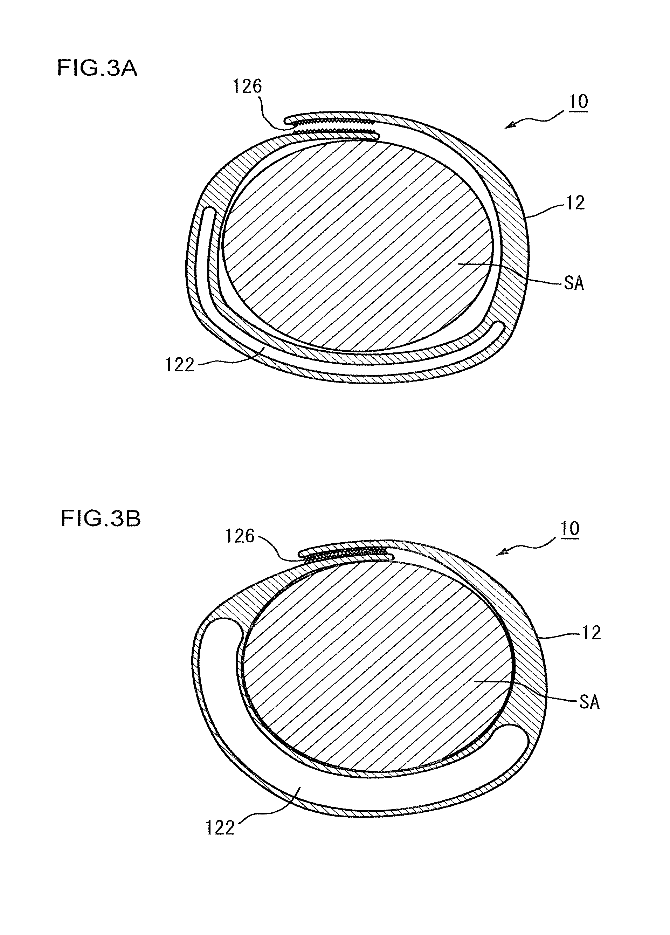

Fig. 3A is a schematic cross-sectional view of the blood pressure measurement unit 10 according to this embodiment attached to the arm SA of a subject, for example the wrist. Fig. 3B is a schematic cross-sectional view showing a state where an oppressing portion 12 of the blood pressure measurement unit 10 is oppressing the arm portion. Hereunder, a blood pressure measurement process according to this embodiment will be described, in which the oppressing portion 12 formed in a shape of a cuff strap that includes an air bag 122 oppresses the arm SA such as the wrist of the subject S, and the blood pressure measurement unit 10 measures the blood pressure by oscillometry.

The oppressing portion 12 according to this embodiment is a strip-shaped bag. The oppressing portion 12 includes a fastener 126 such as a hook-and-loop fastener on the respective end portion in the longitudinal direction. To attach the oppressing portion 12 to the arm SA, the oppressing portion 12 is wound in a loop around the arm SA, the circumferential length is adjusted so as to fit the arm SA, and then the end portions of the oppressing portion 12 are connected via the fastener 126.

As shown in Fig. 2, the blood pressure measurement apparatus 100 according to this embodiment includes the oppressing portion 12 that locally oppresses a body part of the subject S such as the wrist, and the pressure adjustment unit 14 electrically connected to the decision unit 50 and configured to increase or decrease the pressure by which the oppressing portion 12 oppresses the body part. The blood pressure measurement apparatus 100 non-invasively measures the blood pressure from the body part of the subject S such as the wrist, being oppressed by the oppressing portion 12.

The blood pressure measurement apparatus 100 includes the air bag 122 and the air pump 142, the air pump 142 including a pressurizing mechanism 144 that compresses air AIR and supplies the air AIR to the air bag 122. The air AIR supplied by the air pump 142 encompasses all types of gas. The air AIR may be atmospheric air around the blood pressure measurement apparatus 100, or an exclusive gas loaded in a cylinder. The on-off valve 146 is connected to the pressure adjustment unit 14 and the air pump 142, thus constituting a flow path for the air AIR. The internal pressure in the air bag 122 is increased by driving the pressurizing mechanism 144 with the on-off valve 146 closed, and upon opening the on-off valve 146 the air bag 122 is opened to the atmospheric air. The drive control of the pressurizing mechanism 144 and the on/off control of the on-off valve 146 are performed by the pressure adjustment unit 14. The pressure adjustment unit 14 activates and stops the pressurizing mechanism 144 and adjusts the measurement pressure, upon receipt of the start signal for the blood pressure measurement from the decision unit 50. The pressure adjustment unit 14 closes the on-off valve 146 when the blood pressure is to be measured, and opens the on-off valve 146 after the measurement is finished.

The blood pressure measurement unit 10 includes a processing unit 11. The processing unit 11 is a CPU that realizes the pressure adjustment unit 14 and a blood pressure calculation unit 16. The blood pressure calculation unit 16 is configured to calculate the blood pressure of the subject S on the basis of the pulse wave signal PS acquired by a pressure sensor 160.

The blood pressure measurement unit 10 includes the pressure sensor 160, a filter 162, an amplifier 164 and a converter 166. The periodic pulsation of the artery in the arm SA is applied to the air bag 122 in the oppressing portion 12 oppressing the arm SA, and therefore the internal pressure in the air bag 122 periodically varies. While continuously changing the internal pressure in the air bag 122, the amplitude of the internal pressure in the air bag 122 sharply increases or decreases when the internal pressure in the air bag 122 agrees with the systolic blood pressure or the diastolic blood pressure of the subject S. Then the pressure sensor 160 continuously measures the variation waveform (pulse wave signal PS) of the internal pressure in the air bag 122, while the pressure adjustment unit 14 drives the air pump 142 so as to increase or decrease the internal pressure in the air bag 122. In this embodiment, the pulse wave signal PS acquired by the pressure sensor 160 is an analog signal. The filter 162 eliminates high frequency noise and low frequency noise from the pulse wave signal PS acquired by the pressure sensor 160. The amplifier 164 amplifies the pulse wave signal PS from which the noise has been eliminated. The converter 166 is an analog/digital (A/D) converter that converts the amplified pulse wave signal PS into a digital signal. The blood pressure calculation unit 16 analyzes the waveform of the digitized pulse wave signal PS, to thereby calculate the systolic blood pressure and the diastolic blood pressure. Through such a process, the blood pressure measurement unit 10 measures the blood pressure.

To measure the blood pressure, the blood pressure measurement unit 10 may once increase the pressure to the subject S in excess of the systolic blood pressure of the subject S, and then gradually deflate the air bag 122 to thereby measure the systolic blood pressure and the diastolic blood pressure in this order. Alternatively, the blood pressure measurement unit 10 may gradually increase the pressure from below the diastolic blood pressure of the subject S, to thereby measure the diastolic blood pressure and the systolic blood pressure in this order. In this embodiment, it is preferable to adopt the latter method because the maximum pressure only reaches a level close to the systolic blood pressure of the subject S and hence the sleep of the subject S is less likely to be disturbed.

Blood pressure data BP calculated by the blood pressure calculation unit 16 and indicating the blood pressure is transmitted to the control/operation unit 60 through the transmission/reception units 148, 26, and stored in the storage unit 40.

In Figs. 1, 3A and 3B, the pressure adjustment unit 14, the air pump 142, and the on-off valve 146 (hereinafter collectively referred to as pressure control system), the blood pressure calculation unit 16, the pressure sensor 160, the filter 162, the amplifier 164, and the converter 166 (hereinafter collectively referred to as blood pressure signal processing system), and a tube constituting the flow path of the air AIR are not shown. The pressure control system and the blood pressure signal processing system may be formed integrally with the cuff strap (oppressing portion 12). Alternatively, the pressure control system and the blood pressure signal processing system may be provided independent from the cuff strap, and connected thereto through a tube and a signal line. In this case, the pressure control system and the blood pressure signal processing system may be formed integrally with the bio-information acquisition unit 20 so as to share the power source.

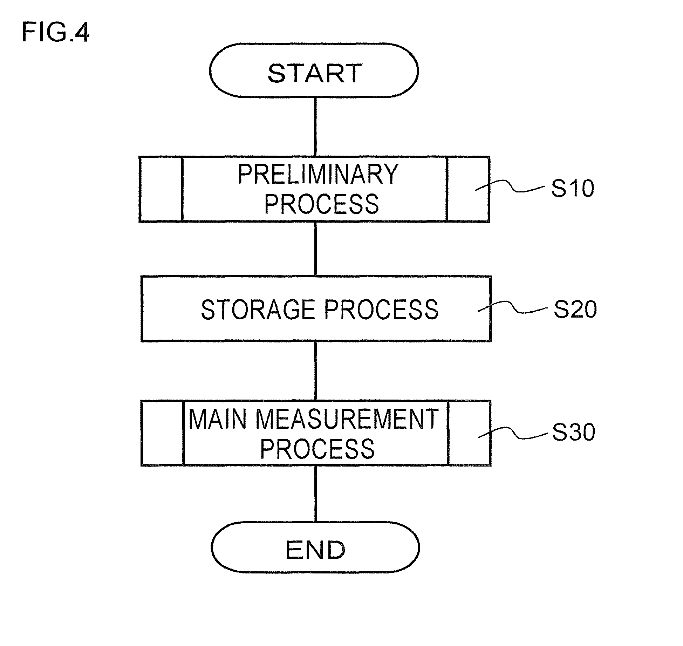

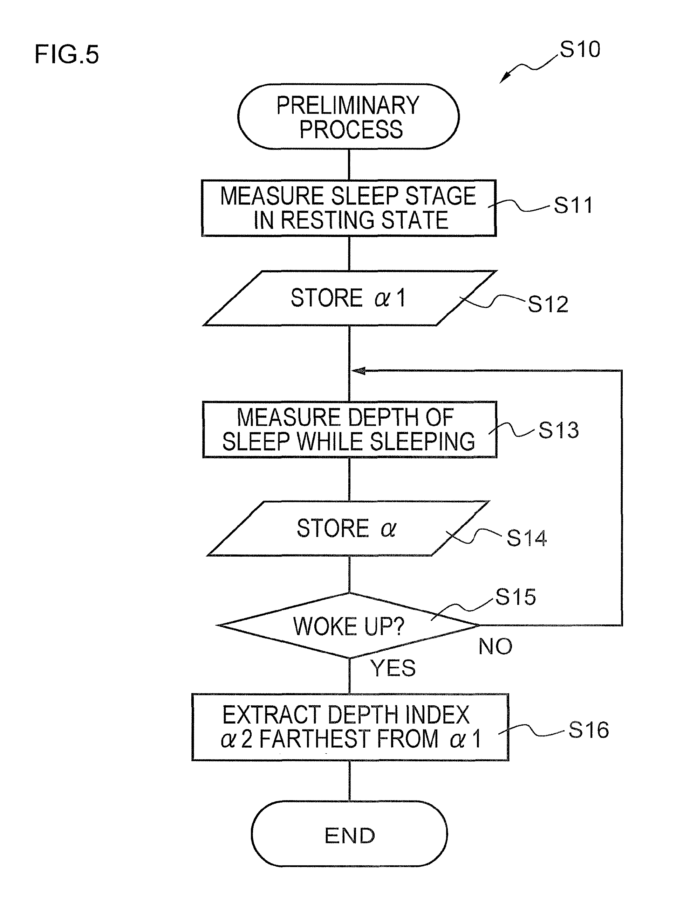

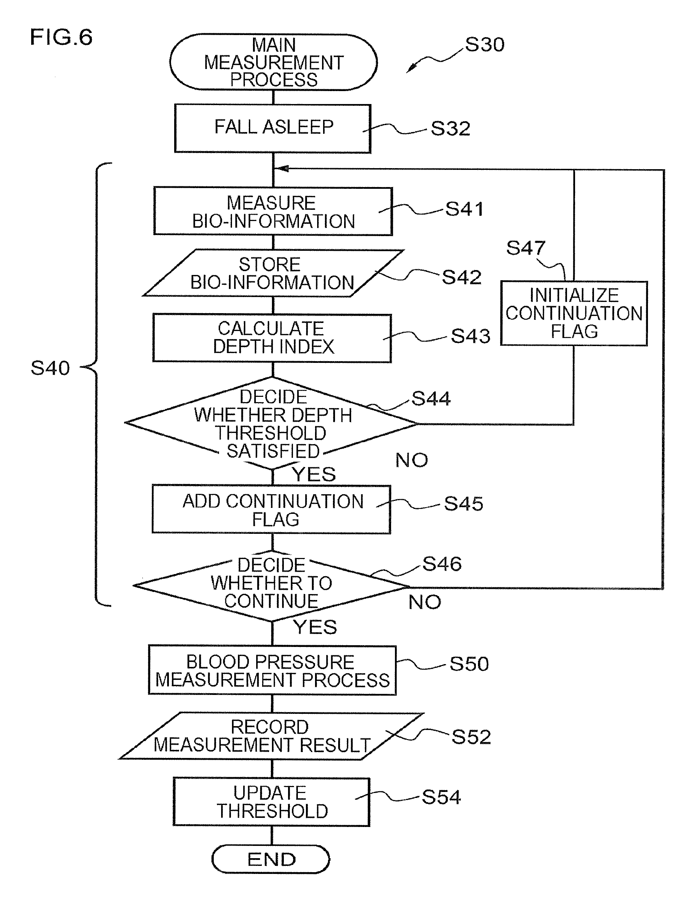

Hereunder, description will be given on a first measurement method of the blood pressure of the subject S performed by the blood pressure measurement apparatus 100 according to this embodiment. Fig. 4 is a flowchart showing a general process of the first blood pressure measurement method. Fig. 5 is a flowchart showing details of the preliminary process S10. Fig. 6 is a flowchart showing details of the main measurement process S30.

First, the outline of the first method will be described.

The first method includes the preliminary process S10, a storage process S20, and the main measurement process S30, as shown in Fig. 4.

In the preliminary process S10, the bio-information acquisition unit 20 acquires the bio-information BI of the subject S, and the depth calculation unit 30 calculates the first depth index $\alpha2$ indicating the sleep stage of the subject S.

In the storage process S2, the control/operation unit 60 stores the calculated first depth index $\alpha2$ in the storage unit 40.

The main measurement process S30 includes a decision process S40 and a blood pressure measurement process S50, as shown in Fig. 6.

The decision process S40 and the blood pressure measurement process S50 are performed in a sleep session different from that of the preliminary process S10. The different sleep session means that the subject S once wakes up and then enters a subsequent sleep session after a predetermined time interval. It is preferable to carry out the decision process S40 and the blood pressure measurement process S50 on a different day from the preliminary process S10.

In the decision process S40, the bio-information acquisition unit 20 acquires the bio-information BI of the subject S in sleep, and the depth calculation unit 30 calculates the second depth index $\alpha$ indicating the sleep stage of the subject S. Then the decision unit 50 decides whether the sleep stage of the subject S in sleep satisfies the predetermined condition (starting condition), on the basis of the first depth index $\alpha2$ stored in the storage unit 40 and the second depth index $\alpha$ calculated by the depth calculation unit 30.

According to the first method, the decision process S40 is repeatedly performed, and when the decision unit 50 decides that the sleep stage of the subject S satisfies the starting condition, the blood pressure measurement process S50 is performed in which the blood pressure measurement unit 10 measures the blood pressure of the subj ect S in sleep.

In the preliminary process S10, a plurality of depth indices are calculated on the basis of the bio-information BI acquired at different time points (epochs). In the storage process S20, the depth index indicating the deepest sleep stage of the subject S among those depth indices is stored in the storage unit 40 as the first depth index $\alpha2$.

The first method will now be described in further details.

As shown in Figs. 2 and 5, in the preliminary process S10 the detection unit 24 acquires the body motion information MV of the subject S about to sleep and lying on the underlay portion 22 of the bio-information acquisition unit 20, and the depth calculation unit 30 in the control/operation unit 60 calculates the depth index $\alpha$. At this point, the sleep stage of the subject S in a resting (awake) state is measured (step S11). The bio-information acquisition unit 20 may acquire the body motion information MV in a single epoch and calculate the depth index $\alpha$, or acquire the body motion information MV and calculate the depth index $\alpha$ from a plurality of epochs, and then calculate the average of the depth indices.

The control/operation unit 60 stores the depth index $\alpha$ thus obtained in the storage unit 40 as the at-rest index $\alpha1$ (step S12).

The bio-information acquisition unit 20 acquires the body motion information MV of the subject S at a predetermined sampling frequency, and analyzes the frequency with respect to each epoch to thereby repeatedly calculate the depth index $\alpha$. After the subject S falls asleep, the depth index $\alpha$ is repeatedly calculated in the same manner (step S13), and the control/operation unit 60 sequentially records the depth indices in the storage unit 40 (step S14). The calculation of the depth index $\alpha$ is continued throughout the night until the subject S wakes up (NO at step S15).

In the preliminary process S10 of the foregoing process, the depth index $\alpha$ is calculated on a real time basis while the subject S is asleep. Instead, the body motion information MV may be recorded in the storage unit 40 while the subject S is asleep, and the frequency analysis of the body motion information MV may be performed for the calculation of the depth index $\alpha$ after the subject S wakes up.

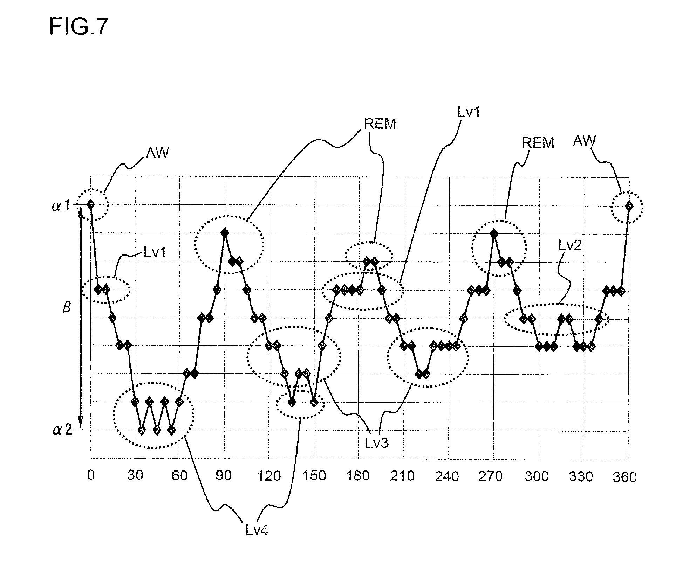

Fig. 7 is a graph showing an example of the sleep characteristics of the subject S obtained through the sampling measurement in the preliminary process S10. The horizontal axis represents the time elapsed after the subject S falls asleep, and the vertical axis represents the sleep stage. The lower positions on the vertical axis indicate the deeper sleep. A code AW denotes an awake state; a code REM denotes a REM sleep; and codes Lv1 to Lv4 denote a non-REM sleep. The codes Lv1 to Lv4 respectively correspond to the level of depth 1 to 4 of the non-REM sleep. In general, the sleep sharply becomes deep after falling asleep and reaches the non-REM sleep (Lv1). The sleep becomes still deeper with the lapse of time, and reaches the deepest sleep stage (Lv4). The deepest sleep continues for approximately 20 to 30 minutes. Then the sleep becomes shallower in a time corresponding to approximately half an ultradian rhythm (sleep cycle; for example 90 minutes), and reaches the REM sleep (REM). Thereafter, the REM sleep and the non-REM sleep are alternately repeated at the ultradian rhythm, and then the subject wakes up (AW).

As a general tendency, the deepest sleep stage of each of the periods experienced according to the ultradian rhythm becomes shallower with the lapse of time. In other words, the sleep stage becomes deepest in the first or second sleep cycle, and the maximal depth of the non-REM sleep becomes shallower in the subsequent sleep cycles, and thus the maximal value of the sleep stage gradually becomes smaller. When the sleep stage assumes the maximum value (deepest value), the subject S is in the deepest and most stable sleep condition. The first method enables the blood pressure in the deep and stable sleep condition, i.e., the basal blood pressure, to be measured.

When the sleep is through and the subject S wakes up (YES at step S15), the control/operation unit 60 adopts the depth index $\alpha$ that satisfies the predetermined starting condition among those depth indices stored in the storage unit 40, as the first depth index $\alpha 2$. According to the first method, the depth index $\alpha$ farthest from the at-rest index $\alpha 1$ is adopted as the first depth index $\alpha 2$ (step S16).

Now, the respiratory rate and the pulse rate during the REM sleep (REM) largely vary among the subjects, and may be significantly higher than the respiratory rate and the pulse rate in a resting state. In contrast, the respiratory rate and the pulse rate during the non-REM sleep are normally lower than the respiratory rate and the pulse rate in a resting state. Accordingly, when setting the first depth index $\alpha 2$, it is preferable to determine the first depth index $\alpha 2$ on the basis of the difference value with a plus or minus sign, between the calculated depth index $\alpha$ and the at-rest index $\alpha 1$, instead of adopting the depth index that makes the largest absolute value of the difference. Such an arrangement prevents the depth index of the REM sleep from being confused with the depth index of the non-REM sleep and erroneously set as the first depth index $\alpha 2$, even when the divergence (absolute value) of depth indices between the REM sleep and resting state is larger than the divergence (absolute value) of depth indices between the resting state and the non-REM sleep. More specific explanation will be given on the assumption that the pulse rate is employed as the depth index. Since normally the pulse rate becomes lower, the deeper the sleep stage in the non-REM sleep is, the depth index in the non-REM sleep is smaller than the at-rest index $\alpha 1$. In other words, the value obtained by (depth index in non-REM sleep - at-rest index $\alpha 1$) is negative. On the contrary, the depth index in the REM sleep is larger than the at-rest index $\alpha 1$, and hence the value obtained by (depth index in REM sleep - at-rest index $\alpha 1$) becomes positive. According to the first method, the depth index corresponding to the deepest sleep stage is set as the first depth index $\alpha 2$. Accordingly, it is preferable to adopt as the first depth index $\alpha 2$ the depth index $\alpha$ with which the difference between the depth index $\alpha$ calculated from time to time from the subject S in sleep and the at-rest index $\alpha 1$, i.e., (depth index $\alpha$ - at-rest index $\alpha 1$), becomes negative and the absolute value of the difference becomes largest, in other words the depth index $\alpha$ that makes the difference smallest.

Hereafter, the depth index $\alpha$ that makes the largest divergence from the at-rest index $\alpha 1$ will be defined as the depth index with which the value obtained by (depth index $\alpha$ - at-rest index $\alpha 1$) has the same sign as (depth index in non-REM sleep - at-rest index $\alpha 1$), and has the largest absolute value.

It is preferable that the depth index has a negative correlation with the level of depth of the sleep stage. In this case, the value obtained by (depth index in non-REM sleep - at-rest index $\alpha 1$) becomes negative, and the depth index that makes the value smallest corresponds to the deepest level of the sleep stage.

Although the REM sleep and the non-REM sleep are distinguished by obtaining the difference value with a plus or minus sign between the depth index $\alpha$ and the at-rest index $\alpha 1$ in the foregoing process, different arrangements may be adopted. For example, the variance of the depth indices in a plurality of closest epochs may be calculated to thereby decide the sleep condition of the subject S, in view of the fact that the depth index in the REM sleep more largely fluctuates with time than in the non-REM sleep. In the case where the variance is smaller than a predetermined threshold, the subject S may be assumed to be in the non-REM sleep. Then the depth index $\alpha$ that makes the absolute value of the difference largest can be properly selected as the first depth index $\alpha 2$, by calculating the difference from the at-rest index $\alpha 1$ only with respect to the depth indices $\alpha$ corresponding to the assumed non-REM sleep of the subject S.

According to the first method, with the first depth index $\alpha 2$ thus determined, the starting condition for the blood pressure measurement can be defined as the state where the sleep stage of the subject S is deepest. Here, a single depth index having the largest divergence from the at-rest index $\alpha 1$ may be extracted and adopted as the first depth index $\alpha 2$, or the average of a plurality of depth indices $\alpha$ having largest divergences may be set as the first depth index $\alpha 2$. Thus, the depth index $\alpha$ that indicates that the sleep stage of the subject S is deepest is extracted, in the first method. In the example shown in Fig. 7, the depth index $\alpha$ corresponding to the deepest sleep stage in the first sleep cycle (maximum value) is extracted as the first depth index $\alpha 2$.

At step S15, the method to recognize that the subject S has woken up is not specifically limited, but various methods may be employed. For example, the subject S may manually operate the I/O unit 28 to turn off the detection unit 24, to thereby notify the measurement apparatus that the subject S has woken up. Alternatively, the subject S may be decided to have woken up when the calculation result of the depth index $\alpha$ given by the depth calculation unit 30 becomes a value corresponding to the awake state.

Referring again to Fig. 4, the at-rest index $\alpha 1$ and the divergence $\beta$ between the first depth index $\alpha 2$ and the at-rest index $\alpha 1$ (= first depth index $\alpha 2$ - at-rest index $\alpha 1$) are stored in the storage unit 40, in the storage process S20. The divergence $\beta$ to be stored may be an absolute value or a value with a plus or minus sign. In this embodiment, the divergence $\beta$ is expressed as an absolute value (= | first depth index $\alpha 2$ - at-rest index $\alpha 1$|).

The main measurement process S30 is carried out with respect to the same subject S as in the preliminary process S10, on a different day or in a different sleep session. As shown in Fig. 6, the depth index $\alpha$ of the subject S in sleep is calculated on a real time basis, in the main measurement process S30 (step S43). When the depth index $\alpha$ (second depth index) comes close to the first depth index $\alpha 2$ it is decided that the starting condition has been satisfied (decision process S40), and the blood pressure measurement is started (blood pressure measurement process S50). More specific description will be given here below.

The subject S operates the I/O unit 28 (see Figs. 1 and 2) to turn on the detection unit 24 of the bio-information acquisition unit 20 to start the measurement of the body motion information MV, before falling asleep (step S32). The bio-information acquisition unit 20 measures the bio-information BI (body motion information MV) of the subject S (step S41), and the control/operation unit 60 records the measured data in the storage unit 40 (step S42). The depth calculation unit 30 performs the frequency analysis of the bio-information BI measured by the bio-information acquisition unit 20 with respect to each epoch, to thereby obtain the power spectrum of the respiratory rate and/or pulse rate. The depth calculation unit 30 calculates the depth index $\alpha$ of the sleep stage on the basis of the power value (step S43).

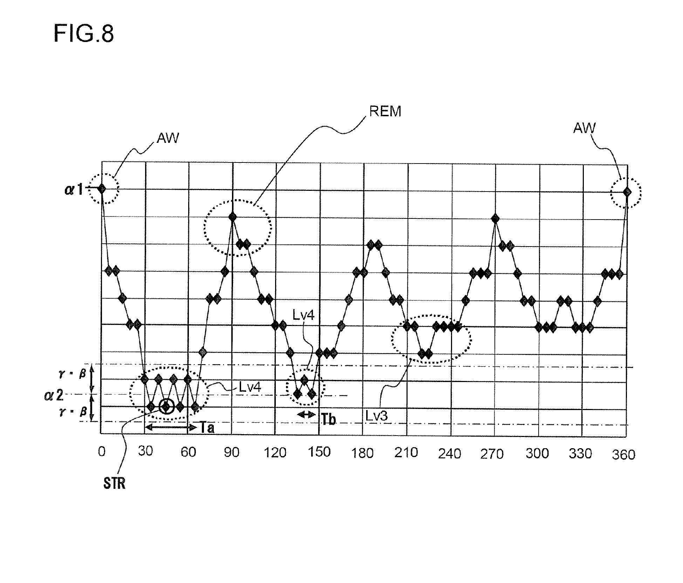

Fig. 8 is a graph showing an example of sleep characteristics of the subject S obtained through the main measurement process S30. The horizontal axis represents the time elapsed after the subject S falls asleep, and the vertical axis represents the sleep stage corresponding to the depth index $\alpha$.

[Decision Method of Starting Condition]

According to the first method, it is decided that the starting condition has been satisfied in the case where the difference between the first depth index $\alpha 2$ stored in the storage unit 40 and the second depth index $\alpha$ calculated by the depth calculation unit 30 on the real time basis has remained below a threshold (depth threshold) through the decision processes S40 continuously repeated for a predetermined period of time.

The difference between the first depth index $\alpha 2$ and the second depth index $\alpha$ may be a value with a plus or minus sign, the absolute value of the difference, or a ratio between the indices. In this embodiment, the absolute value of the difference between the second depth index $\alpha$ and the first depth index $\alpha 2$ (= | second depth index $\alpha$ - first depth index $\alpha 2$|) is adopted as the difference.

The threshold coefficient $\gamma$ is a positive value smaller than 1, stored in the storage unit 40. According to the first method, the value obtained by multiplying the divergence $\beta$ (= | first depth index $\alpha 2$ - at-rest index $\alpha 1$|) calculated in the preliminary process S10 by the threshold coefficient $\gamma$ (= $\gamma \cdot \beta$) is employed as the depth threshold.

In this embodiment, the decision unit 50 decides that the sleep stage of the subject S at the time point when the starting condition is decided to be satisfied is at the deepest level of the non-REM sleep.

The decision unit 50 decides that the starting condition for the blood pressure measurement is satisfied in the case where the sleep stage of the subject S has continuously satisfied the depth condition. Upon deciding that the difference (absolute value) between the newly calculated second depth index $\alpha$ and the first depth index $\alpha 2$ is below the depth threshold (YES at step S44), the decision unit 50 adds a continuation flag indicating that the sleep stage has satisfied the condition (depth condition) (step S45). In other words, in the case where the second depth index $\alpha$ calculated by the depth calculation unit 30 with respect to each epoch satisfies the equation (1) cited here below, the decision unit 50 decides that the sleep stage of the subject S in that epoch has reached the deepest level of the non-REM sleep, and adds the continuation flag.

[Math. 1]

$$\alpha2 - \gamma \cdot \beta \leq \alpha \leq \alpha2 + \gamma \cdot \beta \quad \dots \quad (1)$$

Here, as stated earlier, the variance of the depth indices in a plurality of closest epochs may be calculated to thereby decide the sleep condition of the subject S, in order to prevent the depth index of the REM sleep from being confused with the depth index of the non-REM sleep and erroneously set as the first depth index $\alpha2$. In this case, provided that the subject S is decided to be in the non-REM sleep, the absolute value of the difference between the second depth index $\alpha$ and the first depth index $\alpha2$ may be calculated, to thereby decide that the starting condition is satisfied when the absolute value of the difference is not larger than the depth threshold ($\gamma \cdot \beta$).

In the example shown in Fig. 8, the deepest level (Lv4) of the sleep stages of the subject S is within the range of $\alpha2 \pm \gamma \cdot \beta$.

When the value of the continuation flag reaches a predetermined continuation threshold T1, i.e., when the continuation condition is satisfied, the decision unit 50 decides that the depth condition has been continuously satisfied (YES at step S46). Specifically, for example, the length of an epoch may be set as 30 seconds, and the continuation threshold T1 may be set as 2 to 40. With such settings, the decision unit 50 may decide that the starting condition for the blood pressure measurement is satisfied (YES at step S46), in the case where the depth condition is decided to have been satisfied for 1 to 20 minutes (YES at step S45).

In the case where the value of the continuation flag is below the continuation threshold T1 (NO at step S46), the process returns to the measurement of the bio-information BI in the next epoch (step S41).

On the other hand, in the case where the decision unit 50 decides that the difference between the newly calculated second depth index $\alpha$ and the stored first depth index $\alpha2$ is larger than the depth threshold ($\gamma \cdot \beta$) (NO at step S44), the decision unit 50 initializes the value of the continuation flag (step S47). Then the process returns to the measurement of the bio-information BI in the next epoch (step S41).

In the example shown in Fig. 8, in the first sleep cycle the depth condition is satisfied from 30 to 65 minutes, i.e., for 35 minutes (= Ta), and in the second sleep cycle the depth condition is satisfied from 135 to 145 minutes, i.e., for 10 minutes (= Tb). In the case where, for example, the continuation threshold T1 is set as 30 and the length of an epoch is set as 30 seconds, the continuation condition can be satisfied in the case where the depth condition has been continuously satisfied for 15 minutes. In Fig. 8, the continuation condition is satisfied at 45 minutes (STR) in the first sleep cycle. Thus, the depth condition and the continuation condition are employed in combination to decide whether the starting condition for the blood pressure measurement is satisfied. In the second sleep cycle, Tb is shorter than the threshold and hence the continuation condition is not satisfied. In the third and subsequent sleep cycles, the depth threshold is not reached.

Upon deciding that the starting condition for the blood pressure measurement is satisfied, the decision unit 50 transmits the measurement start signal to the blood pressure measurement unit 10 through the transmission/reception units 26, 148. In the blood pressure measurement unit 10 which has received the measurement start signal, the pressure adjustment unit 14 drives the air pump 142 to pressurize the air bag 122 to thereby oppress the arm SA of the subject S, and the pressure sensor 160 measures the internal pressure in the air bag 122 and acquires the pulse wave signal PS. The blood pressure calculation unit 16 calculates the blood pressure, namely the systolic blood pressure and the diastolic blood pressure on the basis of the pulse wave signal PS which has undergone the aforementioned processings (blood pressure measurement process S50). The measurement result of the blood pressure is recorded in the storage unit 40, through the transmission/reception unit 148, the transmission/reception unit 26, and the control/operation unit 60 (step S52).

By performing the first method, the blood pressure of the subject S can be measured when the sleep stage of the subject S is deepest. The blood pressure thus obtained can be construed as the most stable basal blood pressure of the subject S, which is quite useful for the healthcare management.

In the first method, the thresholds (depth threshold, continuation threshold) may be optionally raised or lowered depending on the decision result in the decision process S40 (threshold update process S54). For example, in the case where it is decided at the step S46 that the starting condition is satisfied, the depth calculation unit 30 calculates the average (average index $\alpha_A$) of the first depth index $\alpha2$ and the depth index indicating the deepest sleep stage (maximum depth index $\alpha3$) among the second depth indices $\alpha$ that have been decided to satisfy the condition. The storage unit 40 stores the average index $\alpha_A$ as a new first depth index $\alpha2$.

Further, the threshold coefficient $\gamma$ stored in the storage unit 40 may be updated to a smaller value.

The average index $\alpha_A$ may be a simple average of the first depth index $\alpha2$ and the maximum depth index $\alpha3$, or a moving average with a weight added to the maximum depth index $\alpha3$. In the first method, the first depth index $\alpha2$

may thus be updated to the average index $\alpha_A$, when the starting condition is satisfied. In this case, even though the first depth index $\alpha2$ indicating an extremely shallow or deep sleep is extracted in the preliminary process S10, the first depth index $\alpha2$ is updated to a value that reflects the sleep characteristics of the subject S by repeating the main measurement process S30 several times. Accordingly, irregularity such that the starting condition is never satisfied during an entire sleep session can be suppressed. Further, reducing the threshold coefficient $\gamma$ in addition to updating the first depth index $\alpha2$ to the average index $\alpha_A$ prevents the satisfaction decision of the depth threshold (step S44) from being excessively loosened. In other words, reducing the threshold coefficient $\gamma$ to thereby lower the depth threshold ($\gamma\cdot\beta$) restricts the result of the depth decision (step S44) from being accepted until the sleep stage of the subject S properly agrees with the average index $\alpha_A$, which leads to improved accuracy of the depth decision.

In the case where the starting condition is never satisfied in the main measurement process S30 during an entire sleep session (NO at step S46), it is preferable to update the threshold coefficient $\gamma$ stored in the storage unit 40 to a larger value. In this case, the first depth index $\alpha2$ stored in the storage unit 40 may be replaced with the depth index $\alpha$ corresponding to the deepest sleep stage in the current sleep session (maximum depth index $\alpha3$), or with the average of the first depth index $\alpha2$ and the depth index $\alpha3$. Such an arrangement increases the probability that the starting condition is satisfied in the subsequent main measurement processes S30.

After the blood pressure is measured in the blood pressure measurement process S50, it is preferable to cancel the operation of the decision process S40. In this case, the blood pressure measurement is not repeated in the same sleep session. In addition, since the deepest sleep condition in each sleep cycle of the same sleep session becomes shallower with time as stated above, it is least likely that the maximum value (deepest value) of the sleep stage is updated in the subsequent sleep cycles, once the blood pressure measurement is performed. Therefore, it is preferable to cancel the decision process S40 to reduce the power consumption of the blood pressure measurement apparatus 100. Further, since the blood pressure measurement is not performed a plurality of times during the same sleep session, the subject S can be saved from disturbance to sleep.

The first method may be modified in various manners.

With the first method, in which the depth threshold and the continuation threshold are employed in combination to decide the starting condition, accidental and momentaneous deep sleep of the subject S, originating from the fluctuation of the sleep stage, can be prevented from being erroneously detected. On the other hand, however, the depth index $\alpha$ may accidentally fail to satisfy the depth condition owing to fluctuation of the bio-information BI, despite the subject S actually having a deep sleep. Accordingly, the following arrangement may be adopted, to prevent the satisfaction decision of the starting condition from becoming excessively strict.

1. In the case where it is decided at the step S44 that the difference between the second depth index $\alpha$ and the first depth index $\alpha2$ is larger than the depth threshold ($\gamma\cdot\beta$), the value of the continuation flag is reduced, instead of being initialized, at the step S47. Such an arrangement allows, despite the depth index $\alpha$ accidentally fluctuating, the starting condition for the blood pressure measurement to be decided on the basis of the depth decision results thus far made. Here, in the case where the continuation flag is zero (initial value), the reduction is not performed.

2. An appropriate threshold coefficient $\gamma$ may be made up on the basis of the sleep characteristics measured in the preliminary process S10. For example, the threshold coefficient $\gamma$ may be determined such that in any of the sleep cycles measured in the preliminary process S10, the depth index $\alpha$ continues to satisfy the foregoing equation (1) for a period twice or more, more preferably three times or more as long as T1. In this case, even though the depth decision is accidentally rejected (NO at step S44) in the non-REM sleep of one of the sleep cycles during the process of repeating the decision (step S46) and the continuation flag is initialized (step S47), repeating the restarted continuation decision (step S46) enables the value of the continuation flag to reach the continuation threshold T1.

Although according to the first method the detection unit 24 acquires the same type of bio-information BI, namely the body motion information MV, in both of the preliminary process S10 and the main measurement process S30, to thereby calculate the depth index $\alpha$, different arrangements may be adopted. The at-rest index $\alpha1$ and the first depth index $\alpha2$ obtained and stored in the storage unit 40 in the preliminary process S10 and the depth index $\alpha$ obtained in the main measurement process S30 may be calculated on the basis of different types of bio-information BI. In other words, the conversion formula between the first depth index $\alpha2$ and the sleep stage may be different from the conversion formula between the first depth index $\alpha2$ and the sleep stage. Further, in the decision process S40, the depth decision may be made through comparison between the sleep stage converted from the first depth index $\alpha2$ and the sleep stage converted from the second depth index $\alpha$, instead of comparing between the depth indices as the equation (1).

Although the deepest sleep stage is employed as the starting condition for the blood pressure measurement in the first method, different arrangements may be adopted. For example, the blood pressure measurement may be activated when the sleep enters the REM sleep stage. In this case, the depth index corresponding to the REM sleep stage (REM) is calculated and stored in the storage unit 40 as the first depth index $\alpha2$, in the preliminary process S10.

In the main measurement process S30, it is preferable to employ in combination the depth condition (first condition) to be satisfied when the second depth index $\alpha$ calculated on the basis of the body motion information MV of the subject S in sleep reaches close to the first depth index $\alpha2$, and a gradual reduction condition (second condition) to be accepted when the sleep stage gradually becomes shallower in the relevant sleep cycle and to the level that satisfiers the depth condition. Then the subject S may be decided to have entered the REM sleep and blood pressure measurement may be activated, when both of the first condition and the second condition are satisfied. Such an arrangement allows the blood pressure of the REM sleep stage to be measured. Thus, not only the value represented by the depth index but also the increasing or decreasing tendency, as well as the increasing/decreasing rate of the sleep stage indicated by the depth index may be utilized for the decision of the starting condition for the blood pressure measurement.

Further, unlike the first method, the measurement of the body motion information MV (step S41) and the calculation of the depth index $\alpha$ (step S43) may still be repeated after the blood pressure measurement is finished, to thereby measure the blood pressure a plurality of times in a single sleep session. Then the respective averages of the systolic blood pressure and the diastolic blood pressure obtained from the plurality of times of measurement may be calculated, and recorded in the storage unit 40 as the basal blood pressure of the subject S.

SECOND EMBODIMENT

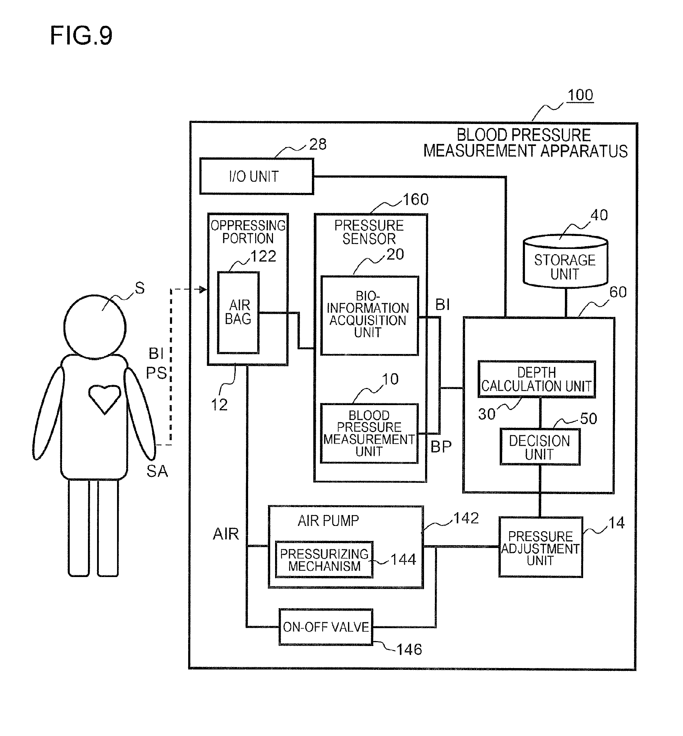

Fig. 9 is a block diagram showing a configuration of the blood pressure measurement apparatus 100 according to a second embodiment.

The blood pressure measurement apparatus 100 according to this embodiment is different from the measurement apparatus 100 according to the first embodiment, in that a cuff strap (oppressing portion 12) is employed to measure both of the bio-information BI and the blood pressure data BP. The configuration of the oppressing portion 12 is the same as that of the first embodiment shown in Figs. 3A and 3B.

The blood pressure measurement apparatus 100 according to this embodiment acquires pulsation information (bio-information BI) indicating the pulse wave pressure of the arm SA of the subject S, through the pressure sensor 160 at a predetermined sampling frequency. Examples of the pulsation information (bio-information BI) include the pulse wave signal PS representing the pressure waveform of the pulse wave, and numerical data of the pulse rate.

The depth calculation unit 30 calculates the depth index $\alpha$ indicating the sleep stage of the subject S, on the basis of the pulsation information. The decision unit 50 decides whether the starting condition for the blood pressure measurement is satisfied, on the basis of the depth index $\alpha$ calculated by the depth calculation unit 30. When the starting condition is satisfied, the blood pressure of the subject S is measured with the pressure sensor 160. Thus, the pressure sensor 160 serves both as the bio-information acquisition unit 20 and as the blood pressure measurement unit 10. In Fig. 9, the blood pressure calculation unit 16, the filter 162, the amplifier 164 and the converter 166 (see Fig. 2) are not shown. In this embodiment, these elements constituting the blood pressure signal processing system are included in the blood pressure measurement unit 10.

The blood pressure measurement apparatus 100 includes the oppressing portion 12 that locally oppresses a body part of the subject S, and the pressure adjustment unit 14 that adjusts the pressure imposed by the oppressing portion 12. The oppressing portion 12 is realized as the cuff strap enclosing therein the air bag 122. The pressure adjustment unit 14, the air pump 142, and the on-off valve 146 (pressure control system) have the same configuration as in the first embodiment (see Fig. 2).

To detect the pulsation information with the pressure sensor 160 (bio-information acquisition unit 20), the air bag 122 is attached to the subject S so as to lightly oppress the arm SA, and the internal pressure in the air bag 122 is measured by the pressure sensor 160. To measure the blood pressure with the pressure sensor 160 (blood pressure measurement unit 10), it is preferable to measure the internal pressure in the air bag 122 with the pressure sensor 160, with the air bag 122 set to oppress the arm SA so as to reach or exceed the systolic blood pressure of the subject S.

Thus, the bio-information acquisition unit 20 according to this embodiment acquires, as the bio-information BI, the pulsation information indicating the pulse (pulse wave) of the body part (arm SA) of the subject S being oppressed by the oppressing portion 12 with a first pressure P1. Then the blood pressure measurement unit 10 measures the blood pressure from the body part (arm SA) being oppressed by the oppressing portion 12 with a second pressure P2 higher than the first pressure P1.

The second pressure P2 varies between a level below the diastolic blood pressure of the subject S and a level equal to or higher than the systolic blood pressure of the subject S. The first pressure P1 is a fixed value set at a level lower than the diastolic blood pressure of an ordinary person.

The general process of the blood pressure measurement method (second method) to be performed by the blood pressure measurement apparatus 100 according to this embodiment, namely the preliminary process S10 and the main measurement process S30, is the same as that of the first embodiment, represented by the flowchart shown in Figs. 4 to 6.

The blood pressure measurement apparatus 100 according to this embodiment is different from the first embodiment in that the bio-information BI is measured (step S41) by oppressing the arm SA. Therefore, in the second method it is preferable to reduce the sampling frequency at which the bio-information BI is measured, for example to 0.2 to 2 times per minute. It is also preferable to measure the bio-information BI for several seconds. Further, it is preferable that, between the finish of the measurement of the bio-information BI and the start of the next measurement of the bio-information BI, the pressure adjustment unit 14 opens the on-off valve 146 to thereby depressurize the air bag 122 of the oppressing portion 12 (reduce the first pressure P1).

In other words, the pressure adjustment unit 14 according to this embodiment reduces the pressure (internal pressure P) of the oppressing portion 12 from the first pressure P1, after the bio-information acquisition unit 20 acquires the pulsation information. Upon receipt of the decision result indicating that the sleep stage of the subject S has satisfied the starting condition from the decision unit 50, the blood pressure measurement unit 10 increases the internal pressure P of the oppressing portion 12 once reduced as above, to the second pressure P2.

In the depressurization of the oppressing portion 12, the internal pressure P in the air bag 122 is reduced to a level close to the atmospheric pressure, so that the pressure to the arm SA substantially becomes zero (non-pressurized state). The non-pressurized state refers to a pressure level that keeps the subject S from perceiving the pressure. The pressure adjustment unit 14 can reduce the internal pressure P in the air bag 122 by opening the on-off valve 146. In addition, the pressure adjustment unit 14 depressurizes the oppressing portion 12 to the non-pressurized state, and suspends the operation of the pressurizing mechanism 144 for at least a part of the interval between the finish of the depressurization and the start of the pressurization. Here, the expression "operation of the pressurizing mechanism 144 is suspended" refers to the state where the pressurizing mechanism 144 is not being driven to pressurize the air bag 122 or to maintain the pressure therein, such as when the pressurizing mechanism 144 is in a stand-by mode or disconnected from the power source. In other words, the mentioned expression refers to the state where the driving components provided in the pressurizing mechanism 144, such as a pressurizing motor or an actuator, are stopped and inaudible. Such an arrangement suppresses the noise of the pressurizing mechanism 144 that may arise during the measurement intervals of the bio-information BI, thereby saving the subject S from disturbance to sleep. At the step S41 in the second method, the pressure adjustment unit 14 closes the on-off valve 146 and turns on the pressurizing mechanism 144 to increase the internal pressure P in the air bag 122 to the first pressure P1, so that the oppressing portion 12 is closely wound around the arm SA (see Fig. 3B). In this state, the pressure sensor 160 acquires the bio-information BI (pulsation information) from the arm SA.

The control/operation unit 60 records the bio-information BI in the storage unit 40 (step S42), and the depth calculation unit 30 calculates the depth index $\alpha$ on the basis of the bio-information BI measured by the bio-information acquisition unit 20 (pressure sensor 160) (step S43). The decision unit 50 decides whether the sleep stage of the subject S satisfies the depth threshold, on the basis of the calculated depth index $\alpha$ (second depth index $\alpha$) and the first depth index $\alpha2$ stored in the storage unit 40 (step S44). In the case where the depth threshold has been continuously satisfied for a predetermined continuation threshold (YES at step S46), the blood pressure measurement unit 10 (pressure sensor 160) measures the blood pressure from the arm SA (step S50). The measurement result of the blood pressure is recorded in the storage unit 40 (step S52), and the first depth index $\alpha2$ may be updated, if desired (threshold update process S54).

According to the second method, the pressure sensor 160 acquires the variation waveform of the internal pressure P in the air bag 122, and decides whether the sleep stage of the subject S satisfies the depth threshold. In the case where the sleep condition close to the maximum level of the sleep stage corresponding to the first depth index $\alpha2$ is maintained for a predetermined period of time, likewise the pressure sensor 160 acquires the variation waveform of the internal pressure P in the air bag 122 and measures the systolic blood pressure and the diastolic blood pressure. Thus, the single cuff strap (oppressing portion 12) is utilized in both of the decision process S40 and the blood pressure measurement process S50, for measuring the basal blood pressure of the subject S.

It is to be understood that the present invention is in no way limited to the foregoing embodiments, but encompasses various modifications and improvements made within the scope of the present invention.

In the blood pressure measurement apparatus 100 according to the first and the second embodiment, the cuff strap is employed for the measurement of the blood pressure of the subject S. The blood pressure measurement with the cuff strap may fail depending on the lying posture of the subject S. Specifically, when the subject S is in a lateral decubitus position with the arm SA wearing the cuff strap (blood pressure measurement unit 10) located under the body, the self weight of the subject S may be superposed on the internal pressure P in the air bag 122, or the cuff strap may be displaced or removed. Accordingly, in the blood pressure measurement process S50 of the first or the second method, it is preferable to detect the measurement failure of the blood pressure on the basis of the up and down profile of the internal pressure P of the oppressing portion 12 controlled by the pressure adjustment unit 14.

Examples of the up and down profile that can be utilized include time duration from the start to the finish of the blood pressure measurement, maximum value of the internal pressure P of the oppressing portion 12 (second pressure P2), increasing rate of the internal pressure P of the oppressing portion 12 (air bag 122) immediately after the activation of the pressurizing mechanism 144 for starting the blood pressure measurement, and the decreasing rate of the internal pressure P of the oppressing portion 12 (air bag 122) immediately after the on-off valve 146 is opened after finishing the blood pressure measurement. In the preliminary process S10, reference profile information of one or a plurality of the foregoing up and down profiles may be acquired and recorded in the storage unit 40, for deciding whether the corresponding up and down profile is within a normal range. Alternatively, the reference profile information may be stored in the storage unit 40 in advance, as information inherent to the blood pressure measurement apparatus 100.

In the blood pressure measurement process S50, the control/operation unit 60 measures the up and down profile of the internal pressure P of the oppressing portion 12, and calculates the divergence from the reference profile information stored in the storage unit 40. In the case where the divergence is larger than a predetermined threshold, the control/operation unit 60 decides that the measurement has failed.

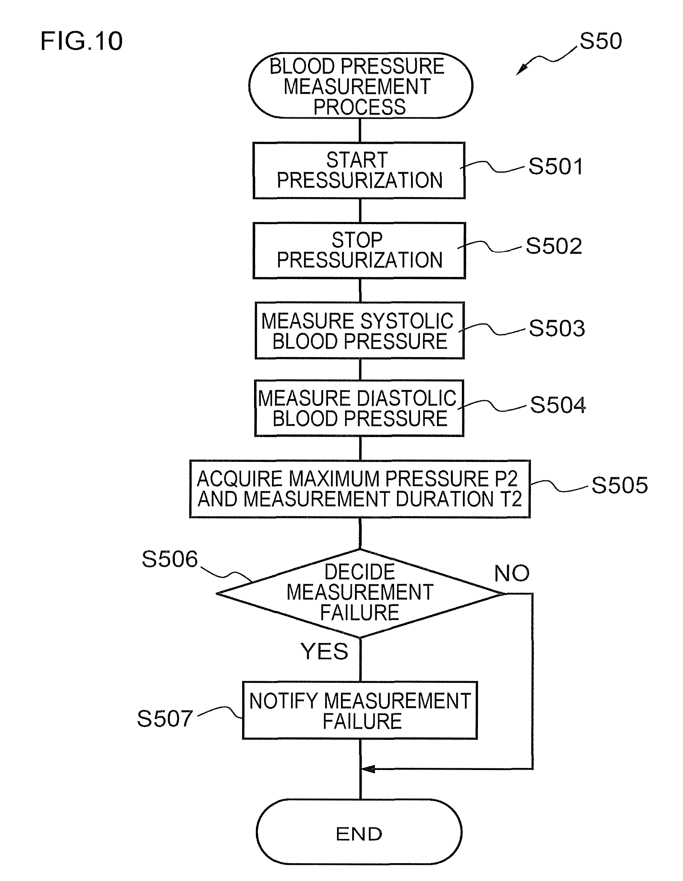

Fig. 10 is a flowchart showing the blood pressure measurement process S50 according to a variation of the embodiments. In this variation, the blood pressure is measured by depressurization. Before starting the blood pressure measurement process S50, the systolic blood pressure of the subject S about to sleep is measured as the preliminary process S10 (see Fig. 4), and a target pressure $P2_0$ of the internal pressure P of the oppressing portion 12 is determined by multiplying the systolic blood pressure by a predetermined coefficient. In addition, the duration from the start to the finish of the blood pressure measurement in the preliminary process S10 (target measurement duration $T2_0$) is measured. The target pressure $P2_0$ and the target measurement duration $T2_0$ are the reference profile information, and stored in the storage unit 40.

In the main measurement process S30, when the decision unit 50 decides that the starting condition for the blood pressure measurement is satisfied (YES at step S46; see Fig. 6), the pressure adjustment unit 14 drives the pressurizing mechanism 144 of the air pump 142 to start to increase the internal pressure P in the air bag 122P (step S501). The pressure adjustment unit 14 stops pressurizing the air bag 122 when the pumping pressure of the air pump 142 reaches the target pressure $P2_0$ (step S502).

Then the pressure adjustment unit 14 slightly opens the on-off valve 146 so as to gradually reduce the internal pressure P in the air bag 122. In the interim, the pressure sensor 160 (blood pressure measurement unit 10) measures the systolic blood pressure and the diastolic blood pressure of the subject S in this order (steps S503, S504). After the measurement of the diastolic blood pressure, the pressure adjustment unit 14 opens the on-off valve 146 to depressurize the air bag 122. The control/operation unit 60 acquires the maximum value of the internal pressure P in the air bag 122P (second pressure P2) measured by the pressure sensor 160 at the step S502 and the duration from the start of the step S501 to the finish of the step S504 (measurement duration T2) (step S505).

The control/operation unit 60 compares between the second pressure P2 and the target pressure $P2_0$, and also between the measurement duration T and the target measurement duration $T2_0$, to thereby decide whether the measurement has failed, according to the respective thresholds (step S506). More specifically, in the case where the second pressure P2 is significantly lower than the target pressure $P2_0$, it is presumed that the cuff strap (oppressing portion 12) is displaced or removed from the arm SA. In the case where the measurement duration T2 is significantly longer than the target measurement duration $T2_0$, it is probable that the subject S is in a lateral decubitus position and the self weight of the subject S is imposed on the cuff strap (oppressing portion 12).

In the case where it is decided that the measurement has failed (YES at step S506), the control/operation unit 60 outputs a message notifying to the effect that the measurement has failed, through the 1/0 unit 28 (step S507). Here, the control/operation unit 60 may output the measured blood pressure data BP through the I/O unit 28, regardless that the measurement failure has been detected. In this case, the subject S can recognize the measurement result of the blood pressure, with the understanding that the accuracy of the blood pressure data BP is not sufficiently high. In the case where the measurement failure has not been detected (NO at step S506), the recording process (step S52; see Fig. 6) is normally performed.

In the case where the measurement failure is detected, the threshold update process S54 (see Fig. 6) may be skipped, or a small weight coefficient may be applied to the second depth index $\alpha$ (maximum depth index $\alpha 3$) that satisfied the starting condition for the blood pressure measurement process S50 that has failed, to thereby calculate an average index $\alpha_A$. More specifically, the depth calculation unit 30 may multiply the first depth index $\alpha 2$ by a first weight coefficient k2, and multiply the maximum depth index $\alpha 3$ by a second weight coefficient k3 smaller than the first weight coefficient k2, to calculate the average index $\alpha_A$. The first weight coefficient k2 and the second weight coefficient k3 are stored in the storage unit 40. Except for the case where the measurement failure is detected, or by calculating, even in such a case, the average index $\alpha_A$ utilizing low weight coefficients, the blood pressure measurement can be carried out with the starting condition that fits the sleep characteristics of each individual subject. Therefore, repeated use of the blood pressure measurement apparatus 100 according to the foregoing embodiments enables more accurate measurement of the basal blood pressure of each subject.

Another variation may be configured as follows. The depth calculation unit 30 repeatedly calculates the sleep stage of the subject S on the basis of the bio-information BI acquired by the bio-information acquisition unit 20. As described above, the sleep stage is a quantified value of the depth of sleep of the subject S. In addition, the predetermined sleep stage (reference sleep stage) indicating the depth of sleep appropriate for the blood pressure measurement is stored in advance in the storage unit 40. For example, the sleep stage corresponding to the non-REM sleep may be adopted as the reference sleep stage. The decision unit 50 decides whether the sleep stage of the subject S has reached the reference sleep stage through comparison between the sleep stage calculated by the depth calculation unit 30 and the reference sleep stage stored in the storage unit 40. Further, the decision unit 50 decides, utilizing the decision result made above, whether the sleep stage of the subject S has remained in the reference sleep stage for a predetermined time (matter of design; for example 1 to 20 minutes). In the case where it is decided that the sleep stage of the subject S has remained in the reference sleep stage for the predetermined time, the decision unit 50 decides that the sleep stage of the subject S satisfies the starting condition for the blood pressure measurement. The remaining aspects may be arranged in the same manner as the first and the second embodiment. This variation eliminates the need to perform the preliminary process of the foregoing embodiment for determining the first depth index and storing the first depth index in the storage unit 40. In addition, according to this variation the blood pressure measurement is activated in the case where the sleep stage has remained in the predetermined state (reference sleep stage) for the predetermined time. In other words, in the case where the sleep stage has remained in the predetermined state for a period shorter than the predetermined time, for example only momentaneously, the blood pressure measurement is not thereby activated. Such an arrangement allows the sleep stage of the subject S to be accurately decided, thereby enabling truly stabilized blood pressure to be accurately measured.

In this variation, the depth calculation unit 30 may calculate the sleep stage by using at least two types of bio-information BI. This leads to increased reliability of the calculation of the sleep stage.

In this variation, further, the depth calculation unit 30 may calculate the sleep stage utilizing the extent of variation of the bio-information BI. This is because generally the biophysical condition of a human largely varies when the sleep is shallow, but only slightly varies when the sleep is deep. For example, the depth calculation unit 30 may calculate at least one of standard deviation, variance, and coefficient of variation from the measured bio-information BI, and calculate the sleep stage on the basis of the calculation result. For example, the depth calculation unit 30 may calculate, each time the bio-information acquisition unit 20 acquires new bio-information BI, the value indicating the extent of variation by using only the measurement values (bio-information BI) most lately acquired within a predetermined range preceding the latest acquisition by the bio-information acquisition unit 20 (for example, within a predetermined length of time before the latest acquisition, or within a predetermined number of acquisition jobs). Calculating thus the sleep stage utilizing the extent of variation of the measurement values of the bio-information BI leads to increased reliability of the calculation of the sleep stage.

For the calculation of the sleep stage utilizing the extend of variation of the measurement values of the bio-information BI, the depth calculation unit 30 may possess in advance the values indicating the extent of variation (standard deviation, variance, and coefficient of variation) and correspondence data showing correspondence between the measurement values and the sleep stage (for example, a table, or a predetermined equation for calculating the sleep stage from the value indicating the variation extent), to thereby calculate the sleep stage utilizing the correspondence data. The correspondence data may be customized for each of the subjects S. For example, the correlation between the extent of variation of the bio-information BI and the depth of sleep may be measured with respect to each subject S utilizing the sleeps preceding the sleep for measuring the blood pressure, to thereby make up the correspondence data, and store such data in the depth calculation unit 30. Such an arrangement leads to increased reliability of the calculation of the sleep stage.

The blood pressure measurement apparatus 100 according to the present invention may be realized as a hardware composed of general-purpose devices such as a central processing unit (CPU), read-only memory (ROM), a random access memory (RAM), and an interface (I/F) unit for reading a computer program and executing the processings specified therein, an exclusive logic circuit configured to execute predetermined processings, or a combination thereof. The constituents of the present invention may be realized in any form provided that the intended function can be achieved, for example as an exclusive hardware that performs a predetermined function, a data processor that performs a predetermined function according to a computer program, a predetermined function realized in a data processor by a computer program, or a desired combination thereof. The constituents of the present invention do not have to be individually independent, but may be configured such that a constituent is a part of another constituent, that a part of a constituent and a part of another constituent overlap, and so forth.

The expression "the constituent (storage unit 40) of the present invention stores data" merely means that the apparatus according to the present invention has the function of storing data therein, and it is not mandatory that the data is stored in the apparatus in advance of the use thereof.

The foregoing embodiments encompass the following technical ideas.

(1) A blood pressure measurement apparatus including a blood pressure measurement unit that measures blood pressure of a subject; a bio-information acquisition unit that continuously acquires bio-information of the subject; a depth calculation unit that repeatedly calculates a depth index indicating a sleep stage of the subject on the basis of the acquired bio-information; a storage unit in which the calculated depth index is stored; and a decision unit that decides whether the sleep stage of the subject in sleep satisfies a predetermined condition, on the basis of a first depth index stored in the storage unit and a second depth index calculated by the depth calculation unit, and the blood pressure measurement unit measures the blood pressure when the decision unit decides that the sleep stage satisfies the condition.

(2) The blood pressure measurement apparatus according to (1) above, in which the first depth index is the depth index indicating a deepest sleep stage among a plurality of depth indices each calculated on the basis of the bio-information acquired at a different time point.

(3) The blood pressure measurement apparatus according to (2) above, in which the decision unit decides that the condition is satisfied in the case where a difference between the calculated second depth index and the first depth index has repeatedly been below a threshold for a predetermined length of time.

(4) The blood pressure measurement apparatus according to (2) or (3) above, in which the depth calculation unit calculates an average value of the first depth index and the second depth index indicating the deepest sleep stage among the second depth indices decided to satisfy the condition, and the storage unit stores the average value as a new one of the first depth index.

(5) The blood pressure measurement apparatus according to any of (1) to (4) above, further including an oppressing unit that locally oppresses a body part of the subject; and a pressure adjustment unit connected to the decision unit and configured to adjust upward and downward a pressure with which the oppressing unit oppresses the body part, the blood pressure measurement apparatus being configured to non-invasively measure the blood pressure from the body part being oppressed.

(6) The blood pressure measurement apparatus according to (5) above, in which the bio-information acquisition unit includes an underlay portion to be placed under the subject in a recumbent position, and a detection unit that acquires body motion information as the bio-infarmatian, the body motion information indicating a body motion of the subject on the basis of the self weight of the subject imposed on the underlay portion.

(7) The blood pressure measurement apparatus according to (5) above, in which the bio-information acquisition unit acquires, as the bio-information, pulsation information indicating pulsation of the subject from the body part being oppressed by the oppressing unit at a first pressure, and the blood pressure measurement unit measures the blood pressure from the body part being oppressed by the oppressing unit at a second pressure higher than the first pressure.

(8) The blood pressure measurement apparatus according to (7) above, in which the pressure adjustment unit reduces the pressure from the first pressure after the bio-information acquisition unit acquires the pulsation information, and increases the reduced pressure to the second pressure upon receipt of a decision result indicating that the sleep stage has satisfied the condition from the decision unit.

(9) The blood pressure measurement apparatus according to (8) above, further including an air bag, and an air pump that pressurizes air by using a pressurizing mechanism and supplies the pressurized air to the air bag, and in which the pressure adjustment unit reduces the pressure to a non-pressurized state and stops operation of the pressurizing mechanism for at least a part of an interval between finishing of the pressure reduction and starting of the pressure increase.

(10) The blood pressure measurement apparatus according to any of (5) to (9) above, configured to detect a measurement failure of the blood pressure, on the basis of an up and down profile of the pressure produced by the pressure adjustment unit.

(11) The blood pressure measurement apparatus according to (4) and (10) above, in which, in the case where the measurement failure has been detected, the depth calculation unit multiplies the first depth index by a first weight coefficient and the second depth index by a second weight coefficient smaller than the first weight coefficient, to thereby calculate the average value.

(12) A method of measuring blood pressure including preliminarily acquiring first bio-information of a subject in sleep thereby calculating a first depth index indicating a sleep stage of the subject; storing the calculated first depth index; acquiring second bio-information of the subject in sleep thereby calculating a second index indicating the sleep stage, the second bio-information being different from the first bio-information; deciding whether the sleep stage satisfies a predetermined condition on the basis of the stored first depth index and the calculated second depth index; and repeating the acquiring, the calculating and the deciding, and measuring blood pressure of the subject upon deciding that the sleep stage satisfies the condition.

(13) The method according to (12) above, in which the step of preliminary acquiring includes calculating a plurality of depth indices on the basis of the bio-information acquired at different time points, and storing, as the first depth index, the depth index indicating the deepest sleep stage among the plurality of depth indices.

(14) The method according to (13) above, further including deciding that the condition is satisfied in the case where a difference between the second depth index and the first depth index has remained below a threshold through the step of deciding repeated for a predetermined length of time.

(15) The method according to (14) above, further including raising and lowering the threshold on the basis of a decision result obtained at the step of deciding.

(16) The method according to any of (12) to (15) above, further including cancelling the step of deciding after the blood pressure is measured.

It is apparent that the present invention is not limited to the above embodiment, and may be modified and changed without departing from the scope and spirit of the invention.

This application claims priority to Japanese Patent Application No. 2012-037479 filed February 23, 2012, and Japanese Patent Application No. 2012-250562 filed November 14, 2012, the entire disclosure of which is incorporated herein by references.

**Claims**

1. A blood pressure measurement apparatus, comprising:

   a blood pressure measurement unit that measures blood pressure of a subject;
   a bio-information acquisition unit that continuously acquires bio-information of the subject;
   a depth calculation unit that repeatedly calculates a depth index indicating a sleep stage of the subject on the basis of the acquired bio-information;
   a storage unit in which the calculated depth index is stored; and
   a decision unit that decides whether the sleep stage of the subject in sleep satisfies a predetermined condition, on the basis of a first depth index stored in the storage unit and a second depth index calculated by the depth calculation unit,
   wherein the blood pressure measurement unit measures the blood pressure when the decision unit decides that the sleep stage satisfies the condition.

2. The blood pressure measurement apparatus according to Claim 1,
   wherein the first depth index is the depth index indicating a deepest sleep stage among a plurality of depth indices each calculated on the basis of the bio-information acquired at a different time point.

3. The blood pressure measurement apparatus according to Claim 2,
   wherein the decision unit decides that the condition is satisfied in the case where a difference between the calculated second depth index and the first depth index has repeatedly been below a threshold for a predetermined length of time.

4. The blood pressure measurement apparatus according to Claim 2 or 3,
   wherein the depth calculation unit calculates an average value of the first depth index and the second depth index indicating the deepest sleep stage among the second depth indices decided to satisfy the condition, and the storage unit stores the average value as a new one of the first depth index.

5. A blood pressure measurement apparatus, comprising:

   a blood pressure measurement unit that measures blood pressure of a subject;
   a bio-information acquisition unit that continuously acquires bio-information of the subject;
   a depth calculation unit that calculates a sleep stage of the subject on the basis of the acquired bio-information;
   a storage unit in which a reference sleep stage indicating a predetermined sleep stage is stored; and
   a decision unit that decides whether the sleep stage of the subject in sleep has remained in the reference sleep stage for a predetermined time, on the basis of the reference sleep stage stored in the storage unit and the sleep stage calculated by the depth calculation unit,
   wherein the blood pressure measurement unit measures the blood pressure when the decision unit decides that the sleep stage of the subject in sleep has remained in the reference sleep stage for the predetermined time.

6. The blood pressure measurement apparatus according to Claim 5,
   wherein the depth calculation unit calculates the sleep stage of the subject on the basis of at least two types of bio-information.

7. The blood pressure measurement apparatus according to Claim 5 or 6,
   wherein the depth calculation unit calculates the sleep stage of the subject on the basis of an extent of variation of the value indicated by the bio-information.

8. The blood pressure measurement apparatus according to Claim 7,
wherein the depth calculation unit calculates the sleep stage of the subject by using correspondence data indicating correlation between the extent of variation of the value indicated by the bio-information and the sleep stage of the subject, the correspondence data being customized for each subject.

9. The blood pressure measurement apparatus according to any of Claims 1 to 8, further comprising:

   an oppressing unit that locally oppresses a body part of the subject; and
   a pressure adjustment unit connected to the decision unit and configured to adjust upward and downward a pressure with which the oppressing unit oppresses the body part,
   the blood pressure measurement apparatus being configured to non-invasively measure the blood pressure from the body part being oppressed.

10. The blood pressure measurement apparatus according to Claim 9,
wherein the bio-information acquisition unit includes an underlay portion to be placed under the subject in a recumbent position, and a detection unit that acquires body motion information as the bio-information, the body motion information indicating a body motion of the subject on the basis of the self weight of the subject imposed on the underlay portion.

11. The blood pressure measurement apparatus according to Claim 9,
wherein the bio-information acquisition unit acquires, as the bio-information, pulsation information indicating pulsation of the subject from the body part being oppressed by the oppressing unit at a first pressure, and
the blood pressure measurement unit measures the blood pressure from the body part being oppressed by the oppressing unit at a second pressure higher than the first pressure.

12. The blood pressure measurement apparatus according to Claim 11,
wherein the pressure adjustment unit reduces the pressure from the first pressure after the bio-information acquisition unit acquires the pulsation information, and
increases the reduced pressure to the second pressure upon receipt of a decision result indicating that the sleep stage has satisfied the condition, from the decision unit.

13. The blood pressure measurement apparatus according to Claim 12, further comprising:

   an air bag; and
   an air pump that pressurizes air by using a pressurizing mechanism and supplies the pressurized air to the air bag, wherein the pressure adjustment unit reduces the pressure to a non-pressurized state and stops operation of the pressurizing mechanism for at least a part of an interval between finishing of the pressure reduction and starting of the pressure increase.

14. The blood pressure measurement apparatus according to any of Claims 9 to 13, configured to detect a measurement failure of the blood pressure, on the basis of an up and down profile of the pressure produced by the pressure adjustment unit.

15. The blood pressure measurement apparatus according to Claim 2, further comprising:

   an oppressing unit that locally oppresses a body part of the subject; and
   a pressure adjustment unit connected to the decision unit and configured to adjust upward and downward a pressure with which the oppressing unit oppresses the body part,
   the blood pressure measurement apparatus being configured to non-invasively measure the blood pressure from the body part being oppressed,
   wherein the depth calculation unit calculates an average value of the first depth index and the second depth index indicating the deepest sleep stage among the second depth indices decided to satisfy the condition, and the storage unit stores the average value as a new one of the first depth index,
   wherein in the case where the measurement failure has been detected, the depth calculation unit multiplies the first depth index by a first weight coefficient and the second depth index by a second weight coefficient smaller than the first weight coefficient, to thereby calculate the average value.

16. A method of measuring blood pressure, comprising:

acquiring as a preliminary process first bio-information of a subject in sleep thereby calculating a first depth index indicating a sleep stage of the subject;

storing the calculated first depth index;

acquiring second bio-information of the subject in sleep thereby calculating a second index indicating the sleep stage, the second bio-information being different from the first bio-information;

deciding whether the sleep stage satisfies a predetermined condition on the basis of the stored first depth index and the calculated second depth index; and

repeating the acquiring, the calculating and the deciding, and measuring blood pressure of the subject upon deciding that the sleep stage satisfies the condition.

17. The method according to Claim 16,

wherein the step of preliminary acquiring includes calculating a plurality of depth indices on the basis of the bio-information acquired at different time points, and storing, as the first depth index, the depth index indicating the deepest sleep stage among the plurality of depth indices.

18. The method according to Claim 17, further comprising deciding that the condition is satisfied in the case where a difference between the second depth index and the first depth index has remained below a threshold through the step of deciding repeated for a predetermined length of time.

19. The method according to Claim 18, further comprising raising and lowering the threshold on the basis of a decision result obtained at the step of deciding.

20. A method of measuring blood pressure, comprising:

measuring blood pressure of a subject;

continuously acquiring bio-information of the subject;

calculating a sleep stage of the subject on the basis of the acquired bio-information; and

deciding whether the sleep stage of the subject in sleep has remained for a predetermined time in a reference sleep stage indicating a predetermined sleep stage, on the basis of the reference sleep stage and the sleep stage calculated in the step of calculating,

wherein the step of measuring blood pressure includes measuring the blood pressure upon deciding that the sleep stage of the subject in sleep has remained in the reference sleep stage for the predetermined time.

21. The method according to any of Claims 16 to 19, further comprising cancelling the step of deciding after the blood pressure is measured.

FIG.1

FIG.2

FIG.3A

126

10

12

SA

122

FIG.3B

126

10

12

SA

122

FIG.4

```
              ┌─────────────┐
              │    START    │
              └─────────────┘
                     │
        ┌────┬────────────────┬────┐
        │    │  PRELIMINARY   │    │        ── S10
        │    │    PROCESS     │    │
        └────┴────────────────┴────┘
                     │
        ┌──────────────────────────┐
        │     STORAGE PROCESS      │        ── S20
        └──────────────────────────┘
                     │
        ┌────┬────────────────┬────┐
        │    │MAIN MEASUREMENT│    │        ── S30
        │    │    PROCESS     │    │
        └────┴────────────────┴────┘
                     │
              ┌─────────────┐
              │     END     │
              └─────────────┘
```

FIG.5

S10

PRELIMINARY
PROCESS

MEASURE SLEEP STAGE
IN RESTING STATE — S11

STORE $\alpha 1$ — S12

MEASURE DEPTH OF
SLEEP WHILE SLEEPING — S13

STORE $\alpha$ — S14

S15
WOKE UP?
NO
YES

EXTRACT DEPTH INDEX
$\alpha 2$ FARTHEST FROM $\alpha 1$ — S16

END

FIG.6

FIG.8

FIG.9

FIG.10

S50

```
        ┌──────────────────────┐
        │   BLOOD PRESSURE     │
        │   MEASUREMENT        │
        │   PROCESS            │
        └──────────────────────┘
                  │
        ┌──────────────────────┐
        │      START           │──── S501
        │  PRESSURIZATION      │
        └──────────────────────┘
                  │
        ┌──────────────────────┐
        │      STOP            │──── S502
        │  PRESSURIZATION      │
        └──────────────────────┘
                  │
        ┌──────────────────────┐
        │ MEASURE SYSTOLIC     │──── S503
        │ BLOOD PRESSURE       │
        └──────────────────────┘
                  │
        ┌──────────────────────┐
        │ MEASURE DIASTOLIC    │──── S504
        │ BLOOD PRESSURE       │
        └──────────────────────┘
                  │
    ┌──────────────────────────────────┐
    │ ACQUIRE MAXIMUM PRESSURE P2       │──── S505
    │ AND MEASUREMENT DURATION T2       │
    └──────────────────────────────────┘
                  │
S506         ◇──────────────◇        NO
        ╱   DECIDE           ╲──────────┐
       ⟨    MEASUREMENT       ⟩          │
        ╲   FAILURE          ╱          │
             ◇──────────────◇           │
                  │ YES                  │
        ┌──────────────────────┐        │
S507────│ NOTIFY MEASUREMENT   │        │
        │ FAILURE              │        │
        └──────────────────────┘        │
                  │                      │
                  │◄─────────────────────┘
                  │
        ┌──────────────────────┐
        │        END           │
        └──────────────────────┘
```

EP 2 630 911 A1

Europäisches Patentamt
European Patent Office
Office européen des brevets

# EUROPEAN SEARCH REPORT

Application Number

EP 13 15 6582

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | EP 1 992 280 A1 (OMRON HEALTHCARE CO LTD [JP]; UNIV JICHI MEDICAL [JP]) 19 November 2008 (2008-11-19) | 1,2,16, 17 | INV. A61B5/022 A61B5/00 |
| Y | * paragraph [0010] - paragraph [0013] * <br> * paragraph [0023] - paragraph [0047] * <br> * paragraph [0089] * <br> * paragraph [0103] - paragraph [0106] * <br> * figures 1,2,4,5 * <br> & JP 2007 229238 A (JICHI MEDICAL UNIV; OMRON HEALTHCARE CO LTD) 13 September 2007 (2007-09-13) | 3-15, 18-21 | |
| Y | US 2006/235315 A1 (AKSELROD SOLANGE [IL] ET AL) 19 October 2006 (2006-10-19) <br> * paragraph [0038] * <br> * paragraph [0052] * <br> * paragraph [0303] * <br> * paragraph [0001] * | 3,5-15, 18-21 | |
| Y | US 2006/293604 A1 (CARLSON GERRARD M [US] ET AL CARLSON GERRARD MERRILL [US] ET AL) 28 December 2006 (2006-12-28) <br> * paragraph [0056] * <br> * paragraph [0061] * <br> * paragraph [0065] * | 4,15,19 | TECHNICAL FIELDS SEARCHED (IPC) <br><br> A61B |
| Y | US 2010/030118 A1 (HIEI TAKEHIKO [JP] ET AL) 4 February 2010 (2010-02-04) | 10 | |
| A | * paragraph [0007] - paragraph [0008] * <br> * paragraph [0040] * <br> * figures 1,2 * | 3-5,14, 15,18-20 | |

-/--

The present search report has been drawn up for all claims

| Place of search | Date of completion of the search | Examiner |
|---|---|---|
| Berlin | 8 May 2013 | Völlinger, Martin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

31

**EUROPEAN SEARCH REPORT**

Application Number

EP 13 15 6582

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | US 2006/200011 A1 (SUZUKI TAKUJI [JP] ET AL) 7 September 2006 (2006-09-07)<br>* paragraph [0049] *<br>* paragraph [0054] *<br>* paragraph [0081] *<br>* paragraph [0115] - paragraph [0116] *<br>* paragraph [0130] - paragraph [0131] *<br>* figures 1,7 *<br>& JP 2006 212218 A (TOSHIBA CORP)<br>17 August 2006 (2006-08-17)<br>----- | 1,16 | |

TECHNICAL FIELDS
SEARCHED        (IPC)

The present search report has been drawn up for all claims

| Place of search | Date of completion of the search | Examiner |
|---|---|---|
| Berlin | 8 May 2013 | Völlinger, Martin |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

...................................................................................

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 13 15 6582

This annex lists the patent family members relating to the patent documents cited in the above-mentioned European search report.
The members are as contained in the European Patent Office EDP file on
The European Patent Office is in no way liable for these particulars which are merely given for the purpose of information.

08-05-2013

| Patent document cited in search report | | | Publication date | Patent family member(s) | | | Publication date |
|---|---|---|---|---|---|---|---|
| EP 1992280 | A1 | | 19-11-2008 | CN | 101394784 | A | 25-03-2009 |
| | | | | CN | 102151129 | A | 17-08-2011 |
| | | | | EP | 1992280 | A1 | 19-11-2008 |
| | | | | JP | 4659646 | B2 | 30-03-2011 |
| | | | | JP | 2007229238 | A | 13-09-2007 |
| | | | | KR | 20080099339 | A | 12-11-2008 |
| | | | | RU | 2008138861 | A | 10-04-2010 |
| | | | | US | 2009234199 | A1 | 17-09-2009 |
| | | | | WO | 2007099775 | A1 | 07-09-2007 |
| US 2006235315 | A1 | | 19-10-2006 | AT | 496577 | T | 15-02-2011 |
| | | | | AU | 2003263571 | A1 | 08-04-2004 |
| | | | | CA | 2499547 | A1 | 01-04-2004 |
| | | | | EP | 1562472 | A1 | 17-08-2005 |
| | | | | EP | 2286723 | A1 | 23-02-2011 |
| | | | | US | 2006235315 | A1 | 19-10-2006 |
| | | | | WO | 2004026133 | A2 | 01-04-2004 |
| US 2006293604 | A1 | | 28-12-2006 | US | 2006293604 | A1 | 28-12-2006 |
| | | | | US | 2011137367 | A1 | 09-06-2011 |
| US 2010030118 | A1 | | 04-02-2010 | CN | 101528127 | A | 09-09-2009 |
| | | | | JP | 4103925 | B1 | 18-06-2008 |
| | | | | JP | 2008142238 | A | 26-06-2008 |
| | | | | US | 2010030118 | A1 | 04-02-2010 |
| | | | | WO | 2008069017 | A1 | 12-06-2008 |
| US 2006200011 | A1 | | 07-09-2006 | JP | 4342455 | B2 | 14-10-2009 |
| | | | | JP | 2006212218 | A | 17-08-2006 |
| | | | | US | 2006200011 | A1 | 07-09-2006 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2012037479 A
- JP 2012250562 A
- JP 2007229238 A
- JP 2006212218 A